# EUROPEAN PATENT SPECIFICATION

(11) **EP 3 277 718 B1**
(45) Date of publication and mention of the grant of the patent: **24.03.2021**
(21) Application number: 16712899.0
(22) Date of filing: 31.03.2016
(51) Int. Cl.: C07K 16/24, A61K 39/00

(54) **DOSAGE REGIMEN FOR ANTI-MIF ANTIBODIES**
DOSIERUNGSSCHEMA FÜR ANTI-MIF-ANTIKÖRPER
RÉGIME POSOLOGIQUE DES ANTICORPS ANTI-MF

(30) Priority: 31.03.2015 US 201562141190 P; 18.05.2015 US 201562163267 P
(43) Date of publication of application: 07.02.2018
(73) Proprietor: Baxalta GmbH, 8152 Glattpark (Opfikon) (CH); Baxalta Incorporated, Bannockburn, IL 60015 (US)
(72) Inventor: KERSCHBAUMER, Randolf, 6300 Zug (CH); THIELE, Michael, 1030 Wien (AT); DOUILLARD, Patrice, 1020 Wien (AT)
(74) Representative: Hoffmann Eitle
(86) International application number: PCT/EP2016/057055
(87) International publication number: WO 2016/156489

(56) References cited:
- WO-A1-98/17314
- WO-A1-2013/156473
- WO-A2-01/64749
- WO-A2-02/067862
- F. HUSSAIN ET AL: "Human Anti-Macrophage Migration Inhibitory Factor Antibodies Inhibit Growth of Human Prostate Cancer Cells In Vitro and In Vivo", MOLECULAR CANCER THERAPEUTICS, vol. 12, no. 7, 1 July 2013 (2013-07-01), pages 1223-1234, XP55199203, ISSN: 1535-7163, DOI: 10.1158/1535-7163.MCT-12-0988
- MEYER-SIEGLER KATHERINE L ET AL: "Inhibition of macrophage migration inhibitory factor decreases proliferation and cytokine expression in bladder cancer cells", BMC CANCER, BIOMED CENTRAL, LONDON, GB, vol. 4, no. 1, 12 July 2004 (2004-07-12), page 34, XP021004633, ISSN: 1471-2407, DOI: 10.1186/1471-2407-4-34
- SHIMIZU T ET AL: "HIGH EXPRESSION OF MACROPHAGE MIGRATION INHIBITORY FACTOR IN HUMAN MELANOMA CELLS AND ITS ROLE IN TUMOR CELL GROWTH AND ANGIOGENESIS", BIOCHEMICAL AND BIOPHYSICAL RESEARCH COMMUNICATIONS, ACADEMIC PRESS INC. ORLANDO, FL, US, vol. 264, no. 3, 2 November 1999 (1999-11-02), pages 751-758, XP001008703, ISSN: 0006-291X, DOI: 10.1006/BBRC.1999.1584

## Description

The present invention pertains to anti-MIF antibodies in the treatment of cancer, administered using a dosage regimen.

### BACKGROUND

Macrophage migration inhibitory factor (MIF) is a cytokine initially isolated based upon its ability to inhibit the *in vitro* random migration of peritoneal exudate cells from tuberculin hypersensitive guinea pigs (containing macrophages) (Bloom et al. Science 1966, 153, 80-2; David et al. PNAS 1966, 56, 72-7). Today, MIF is known as a critical upstream regulator of the innate and acquired immune response that exerts a pleiotropic spectrum of activities.

The human MIF cDNA was cloned in 1989 (Weiser et al., PNAS 1989, 86, 7522-6), and its genomic localization was mapped to chromosome 22. The product of the human MIF gene is a protein with 114 amino acids (after cleavage of the N-terminal methionine) and an apparent molecular mass of about 12.5 kDa. MIF has no significant sequence homology to any other protein. The protein crystallizes as a trimer of identical subunits. Each monomer contains two antiparallel alpha-helices that pack against a four-stranded beta-sheet. The monomer has additional two beta-strands that interact with the beta-sheets of adjacent subunits to form the interface between monomers. The three subunits are arranged to form a barrel containing a solvent-accessible channel that runs through the center of the protein along a molecular three-fold axis (Sun et al. PNAS 1996, 93, 5191-5196).

It was reported that MIF secretion from macrophages was induced at very low concentrations of glucocorticoids (Calandra et al. Nature 1995, 377, 68-71). However, MIF also counter-regulates the effects of glucocorticoids and stimulates the secretion of other cytokines such as tumor necrosis factor TNF-α and interleukin IL-1 β (Baugh et al., Crit Care Med 2002, 30, S27-35). MIF was also shown e.g. to exhibit pro-angiogenic, pro-proliferative and anti-apoptotic properties, thereby promoting tumor cell growth (Mitchell, R.A., Cellular Signalling, 2004. 16(1): p. 13-19; Lue, H. et al., Oncogene 2007. 26(35): p. 5046-59). It is also e.g. directly associated with the growth of lymphoma, melanoma, and colon cancer (Nishihira et al. J Interferon Cytokine Res. 2000, 20:751-62).

MIF is a mediator of many pathologic conditions and thus associated with a variety of diseases including *inter alia* inflammatory bowel disease (IBD), rheumatoid arthritis (RA), acute respiratory distress syndrome (ARDS), asthma, glomerulonephritis, IgA nephropathy, myocardial infarction (MI), sepsis and cancer, though not limited thereto.

Polyclonal and monoclonal anti-MIF antibodies have been developed against recombinant human MIF (Shimizu et al., FEBS Lett. 1996; 381, 199-202; Kawaguchi et al, Leukoc. Biol. 1986, 39, 223-232, and Weiser et al., Cell. Immunol. 1985, 90, 167-78).

Anti-MIF antibodies have been suggested for therapeutic use. Calandra et al., (J. Inflamm. (1995); 47, 39-51) reportedly used anti-MIF antibodies to protect animals from experimentally induced gram-negative and gram-positive septic shock. Anti-MIF antibodies were suggested as a means of therapy to modulate cytokine production in septic shock and other inflammatory disease states.

US 6,645,493 discloses monoclonal anti-MIF antibodies derived from hybridoma cells, which neutralize the biological activity of MIF. It could be shown in an animal model that these mouse-derived anti-MIF antibodies had a beneficial effect in the treatment of endotoxin-induced shock.

US 200310235584 discloses methods of preparing high affinity antibodies to MIF in animals in which the MIF gene has been homozygously knocked-out.

PCT/EP2013/057894 (published as WO2013/156473) discloses anti-MIF antibodies and their uses in combination with chemotherapeutic agents in the treatment of cancer.

Glycosylation-inhibiting factor (GIF) is a protein described by Galat et al. (Eur. J. Biochem, 1994, 224, 417-21). MIF and GIF are now recognized to be identical. Watarai et al. (PNAS 2000, 97, 13251-6) described polyclonal antibodies binding to different GIF epitopes to identify the biochemical nature of the posttranslational modification of GIF in Ts cells. Watarai et al, *supra,* reported that conformational changes of GIF can be induced by cystinylation *in vitro.* One type of change occurs by chemical modification of a single cysteine residue. The chemical modification leads to conformational changes within the GIF protein.

One of those medical diseases and disorders in which MIF has been implicated for the past decades is - as pointed out above - cancer. Cancer is generally treated via various routes, one of them being the use of so-called chemotherapeutic agents (which are the basis of anticancer chemotherapy). The concept underlying chemotherapy in the general sense thereof, posits that a disease or disorder (caused by bacteria, viruses, parasites and cancer cells) can be effectively treated by way of chemical compounds. One particular indication of chemotherapy is cancer. Chemotherapeutic agents can act for example by killing cells that divide more rapidly than other cells, and thus target cancer cells which commonly divide more rapidly than non-cancerous cells. Most chemotherapeutic agents drugs work by impairing cell division, i.e., they act at one or several stages of the cell cycle and thus are able to target cells that divide more rapidly. Chemotherapeutic agents can be either cytostatic, i.e. they slow down or abrogate the growth or division of cells; other chemotherapeutic drugs can cause damage to cells and kill them; in that case they are termed cytotoxic. Most cytotoxic drugs inflict a damage that *per se* does not suffice to kill a cell but that generates a stimulus to initiate programmed cell death (apoptosis).

In general, major classes of chemotherapeutic drugs are alkylating agents, anti-metabolites, anthracyclines, plant alkaloids, topoisomerases and other anti-tumour agents. Most commonly, as mentioned above, these drugs affect cell division; they can also affect DNA synthesis or function. Other chemotherapeutics do not directly interfere with DNA. These are newer classes of chemotherapeutic agents, which are referred to as signal interceptors, which include monoclonal antibodies and tyrosine kinase inhibitors like imatinib mesylate. Examples for alkylating agents, which alkylate nucleophilic functional groups are mechlorethamine, cyclophosphamide, chlorambucil, melphalane, trofosfamide, ifosfamide, carmustine, lomustine, dacarbazine, temozolomide, mitomycine C and many others. Cisplatin, carboplatin, oxaliplatin and other platinum containing compounds form stable complexes with DNA.

Cytotoxic anti-metabolites are folic acid analogues (e.g., methotrexate/aminopterin, raltitrexed, pemetrexed, or leucovorin (also termed folinic acid)), purines (e.g., 6-mercaptopurine, azathioprine, thioguanine, fludarabine, cladribine) or pyrimidines (cytarabine, gemcitabine, deazacytidine, 5-fluoruracil and its prodrugs including capecitabine), Antimetabolites either inhibit DNA-synthesis by interfering with crucial steps in the *de novo* synthesis of purine and pyrimidine nucleotides or they become incorporated into DNA during the S-phase of the cell cycle, where they interfere with DNA-folding, DNA-repair or methylation. Alternatively, some compounds also become incorporated into RNA.

Examples for alkaloids and terpenoids which are derived from plants and block cell division by preventing microtubule function are vinca-alkaloids and taxanes. Particularly well known vinca-alkaloids are vincristine, vinblastine, vinorelbine and vindesine. Podophyllotoxin is an additional example of a plant-derived compound. An example for a taxane is docetaxel or paclitaxel. Estramustin is an example of a synthetic compound that targets tubulin.

Examples of topoisomerase inhibitors, which are inhibitors of enzymes that maintain the topology of DNA, include camphtotecines like irinotecan and topotecan (type 1 topoisomerase inhibitors) or amsacrin, etoposide, etoposide phosphate and teniposide (topoisomerase-type 2 inhibitors).

Finally, examples of antineoplastic intercalating agents include dactinomycin, doxorubicin, epirubicin, bleomycin and others.

A comprehensive overview is comprised in Goodman and Gilman, The Pharmacological Basis of Therapeutics, 12th Edition, "General Principles of Cancer Chemotherapy. Introduction" as shown below.

Several tumors are susceptible to hormone therapy: glucocorticoids (e.g., prednisolone, dexamethasone and may others) promote apoptosis of lymphoma cells. They are therefore included in typical chemotherapeutic regimen. Similarly, several types of cancer are susceptible to hormonal interventions. This includes, e.g., breast cancer, ovarian cancer and prostate cancer. Hormonal ablation can be achieved by suppressing pituitary release of gonadotropins with gonadotropin-releasing hormone receptor agonists (e.g., buserelin, goserelin, leuprolide, hisrelin etc.), which induce desensitization of the receptor and hence inhibit hormone production, or with gonadotropin-releasing hormone receptor antagonists (e.g., degarelix). Altematively, the action of estrogens and of androgens may be blocked by hormone receptor antagonists: compounds that act as partial agonists at estrogen receptors (also referred to as selective estrogen receptor modulators, SERM's) include tamoxifen, raloxifen and toremifen. Fulvestrant is an example of a pure estrogen recepor antagonist. Androgen receptors can be blocked by antagonists such as flutamide, bicalitamide and cyproterone. Finally, hormonal ablation can be achieved by blocking the pertinent enzymes, which are responsible for their synthesis. In the case of estrogens, it is the aromatase (CYP19), which is blocked by compounds such as aminoglutethimide, formestane, exemestane, anastrazole and letrozole. Androgen production can be suppressed by inhibiting the enzyme 17 α-hydroxylase/C17,20 lyase (CYP17A1) with abiraterone. Regardless of by which approach hormonal input is blocked, the growth of susceptible cancer cells is suppressed and their apoptosis is promoted.

Although chemotherapeutic agents have been shown to be useful and successful in the treatment of several different cancer types, chemotherapeutic regimen have a range of side effects, depending on the type of medication used. Most common side effects include depression of the immune system which can result in potentially fatal infections, fatigue, anaemia, a tendency to bleed easily, gastrointestinal distress, like nausea and vomiting, diarrhoea or constipation and hair loss. Further, damage to specific organs may occur, which results e.g. in heart damage, liver damage, kidney damage, damage to the inner ear, damage to the peripheral nervous system and brain dysfunction.

All of these side effects increase severely if the dosage of the given chemotherapeutic drug is augmented. Decreasing the amount of the drug administered will usually also result in lower incidence and/or alleviated side effects; however, it will normally also reduce the efficacy of the treatment.

Thus, there remains a need in the art for the provision of a therapy of cancer which allows to improve the treatment with an anti-MIF antibody.

### DESCRIPTION OF THE INVENTION

This object has been solved by the present invention.

In particular, the present inventors have found that surprisingly, when the dosage of anti-MIF antibodies is increased, the efficacy of the cancer treatment for solid tumors follows a non-monotonic curve. In particular, it will reach a maximum value already at dosages of 1-10 mg per kg body weight of anti-MIF antibodies in humans, Additionally, it was found that the pharmacokinetic properties of anti-MIF antibodies including their terminal half-life are more favourable in primates compared to mice. Thus, according to the present invention, anti-MIF antibodies can be administered to humans at the dosages of the invention as indicated below. Thus, the present invention provides an advantageous dosage regimen for anti-MIF antibodies, which allows to administer anti-MIF antibodies at the dosages of the invention as indicated below, while maximizing the efficacy of the cancer treatment.

Elevated MIF levels, i.e. elevated levels of MIF in genera! are detected after the onset of various diseases, *inter alia* after the onset of cancer. However, MIF circulates also in healthy subjects, which makes a clear differentiation difficult. oxMIF, on the contrary, has, in general, not been detected in healthy tissue of subjects. It has been discovered after thorough research of MIF and antibodies thereto that the antibodies RAB9, RAB4 and RAB0, as well as RAM9, RAM4 and RAM0, specifically bind to oxMIF (and are incapable of binding to redMIF).

In earlier experiments carried out by the inventors, it could be shown that oxidative procedures like cystine-mediated oxidation, GSSG (ox. Glutathione)-mediated oxidation or incubation of MIF with Proclin®300 or protein crosslinkers (e.g. BMOE) causes binding of MIF to the above mentioned antibodies.

This additional knowledge regarding (ox)MIF served as a basis for the further studies of the present inventors.

Thus, the invention is as defined by the appended claims.

The above-mentioned antibodies are characterized and supported by both their sequences as well as by deposits as plasmids in *E*.*coli*, comprising either the light or the heavy chain of each of the above mentioned antibodies RAB0, RAB4 and RAB9, respectively, as well as RAM0, RAM4 and RAM9, respectively,

The plasmids are characterized by their DSM number which is the official number as obtained upon deposit under the Budapest Treaty with the German Collection of Microorganisms and Cell Cultures (DSMZ), Mascheroder Weg 1b, Braunschweig, Germany. The plasmids were deposited in *E. coli* strains, respectively. The plasmid with the DSM 25110 number comprises the light chain sequence of the anti-MIF antibody RAB4. The plasmid with the DSM 25112 number comprises the heavy chain (IgG4) sequence of the anti-MIF antibody RAB4.

The co-expression of plasmids DSM 25110 and DSM 25112 in a suitable host cell results in the production of preferred anti-MIF antibody RAB4.

The plasmid with the DSM 25111 number comprises the light chain sequence of the anti-MIF antibody RAB9. The plasmid with the DSM 25113 number comprises the heavy chain (IgG4) sequence of the anti-MIF antibody RAB9.

The co-expression of plasmids DSM 25111 and DSM 25113 in a suitable host cell results in the production of preferred anti-MIF antibody RAB9.

The plasmid with the DSM 25114 number comprises the light chain sequence of the anti-MIF antibody RAB0. The plasmid with the DSM 25115 number comprises the heavy chain (IgG4) sequence of the anti-MIF antibody RAB0.

The co-expression of plasmids DSM 25114 and DSM 25115 in a suitable host cell results in the production of preferred anti-MIF antibody RAB0.

Similarly, the light and heavy chains of RAM0, RAM9 and RAM4 have been similarly deposited under the Budapest Treaty on April 12, 2012 with the DSMZ, Braunschweig, Germany. The following designations have been used:
RAM9 - heavy chain: E.coli GA.662-01,pRAM9hc - DSM 25860.
RAM4 - light chain: E.coli GA.906-04,pRAM4Ic - DSM 25861.
RAM9 - light chain: E.coli GA.661-01.pRAM9lc - DSM 25859.
RAM4 - heavy chain: E.coli GA.657-02-pRAM4hc - DSM 25862.
RAM0 - light chain: E.coli GA.906-01.pRAM0lc - DSM 25863.
RAM0 - heavy chain: E.coli GA.784-01.pRAM0hc - DSM 25864.

The term "prophylactic" or "therapeutic" treatment is art-recognized and refers to administration of a drug to a subject. If it is administered prior to clinical manifestation of the unwanted condition (e.g., disease or other unwanted state of the subject) then the treatment is prophylactic, i.e., it protects the subject against developing the unwanted condition, whereas if administered after manifestation of the unwanted condition, the treatment is therapeutic (i.e., it is intended to diminish, ameliorate or maintain the existing unwanted condition or side effects therefrom).

The cancer treatment according to the invention is therapeutic. The therapeutic cancer treatment according to the invention includes treatments to diminish, ameliorate or maintain the cancer, That is, the cancer therapeutic treatment according to the invention also includes maintenance therapy. It also includes palliative treatment in accordance with the meaning of the term "palliative treatment" as known in the art.

The terms "treatment that maintain(s)" or "maintenance therapy" as used herein are to be understood in accordance with the common meaning of the term "maintenance therapy" as known in the art.

The cancer treatment according to the invention can be a first-line therapy, a second-line therapy or a third-line therapy or beyond. The meaning of these first-, second- or third-line therapies is in accordance with the terminology that is commonly used by the US National Cancer Institute. Thus, a "first-line therapy" that is given to the human subject is the first treatment for the cancer. A "second-line therapy" that is given to the human subject is a treatment that is given when the initial treatment (first-line therapy) does not work, or stops working. A "third-line therapy" that is given to the human subject is a treatment that is given when both initial treatment (first-line therapy) and subsequent treatment (second-line therapy) do not work, or stop working. The distinction of whether or not a treatment is working is made based on the RECIST criteria for the evaluation of a treatment response of solid tumors and/or based on clinical progression. The RECIST criteria are known to the person skilled in the art and have been published in Eisenhauer et al., European Journal of Cancer 45 (2009), 228-247. In a preferred embodiment, the treatment according to the invention is a second-line therapy or a third-line therapy. In a more preferred embodiment, the treatment according to the invention is a third-line therapy.

As used herein an anti-(ox)MIF compound refers to any agent that attenuates, inhibits, opposes, counteracts, or decreases the biological activity of (ox)MIF. An anti(ox)MIF compound may be an agent that inhibits or neutralizes (ox)MIF activity, for example an antibody, particularly preferred, the antibodies as described herein, even more preferred the antibodies RAB9, RAB4 and/or RAB0. Very preferred antibodies are RAM9, RAM4 and/or RAM0.

The preferred MIF antagonist in accordance with the present invention is an anti-MIF antibody. The anti-MIF antibody of the invention is an antibody against oxMIF which does not bind to MIF in its reduced state. In other embodiments, the anti-oxMIF antibodies, e.g., the antibodies mentioned above or an antigen-binding portion thereof bind oxMIF with a K_{D} of less than 100 nM, preferably a K_{D} of less than 50 nM, even more preferred with a K_{D} of less than 10nM.

Very preferred, the antibodies bind to oxMIF with a K_{D} of less than 5 nM.

The invention further relates to kits comprising an anti-MIF antibody or an antigen-binding portion thereof in the dosages of the invention as well as optionally a chemotherapeutic agent according to the invention. A kit may include in addition to the antibody and optionally the chemotherapeutic agent, further therapeutic agents and uses thereof. A kit also can include instructions for use in a therapeutic method. The dosages comprised by the kit of the invention can be packaged in any form which is suitable for administration. Preferably, the dosages comprised by the kit of the invention are packaged in the form of vials or pre-filled syringes. More preferably, the dosages comprised by the kit of the invention are packaged in the form of pre-filled syringes which are adapted for administration of a single dose.

Earlier results have shown that an anti-MIF antibody that only binds oxMIF and does not bind redMIF and further inhibits GCO and/or cell proliferation induces a beneficial effect in an animal model.

### DETAILED DESCRIPTION OF THE INVENTION

The invention is further described in the figures as enclosed.

### Description of the figures:

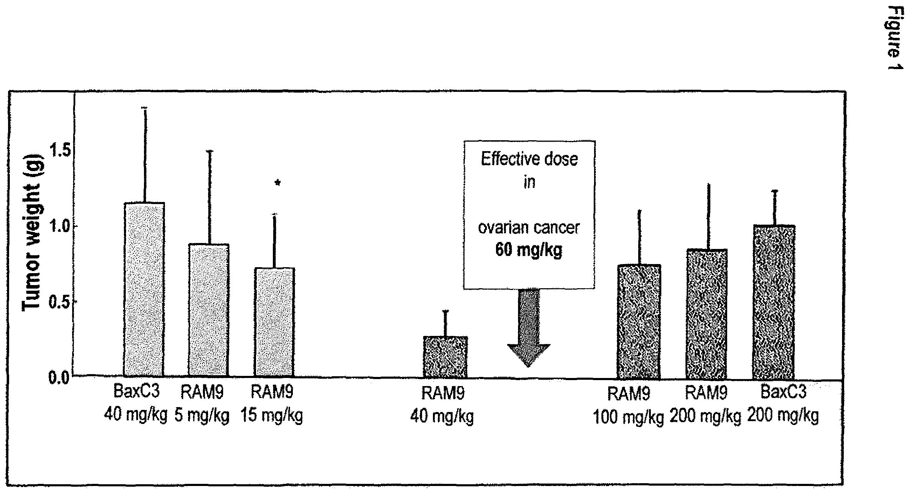

- **Figure 1**:: Dose response study for the RAM9 antibody in a prostate cancer xenograft model. PC-3 cells were injected into the flank of MF nude mice using matrigel. The study was conducted using a preventive setup comprising i.p. injection of antibody every other day. The results are shown in the Figure. The Figure also indicates the effective dosage for an ovarian cancer model by an arrow. The BaxC3 antibody served as a control antibody.

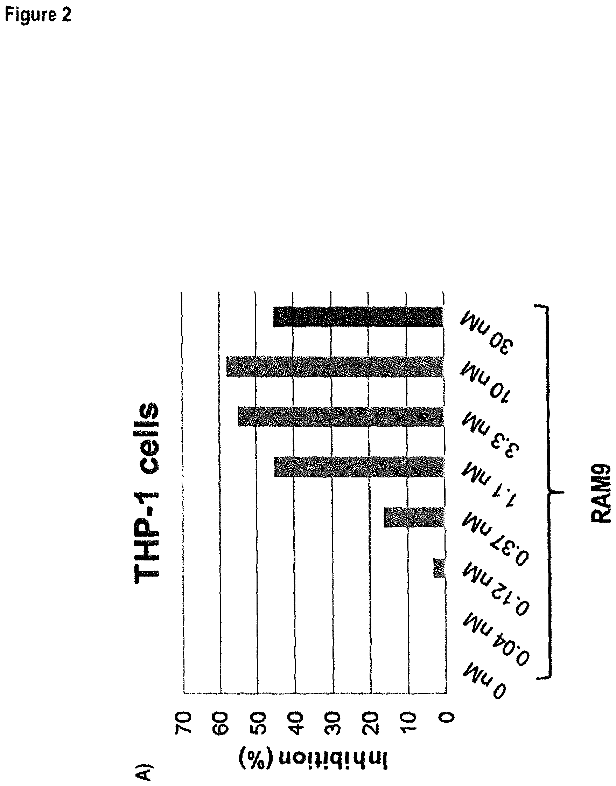

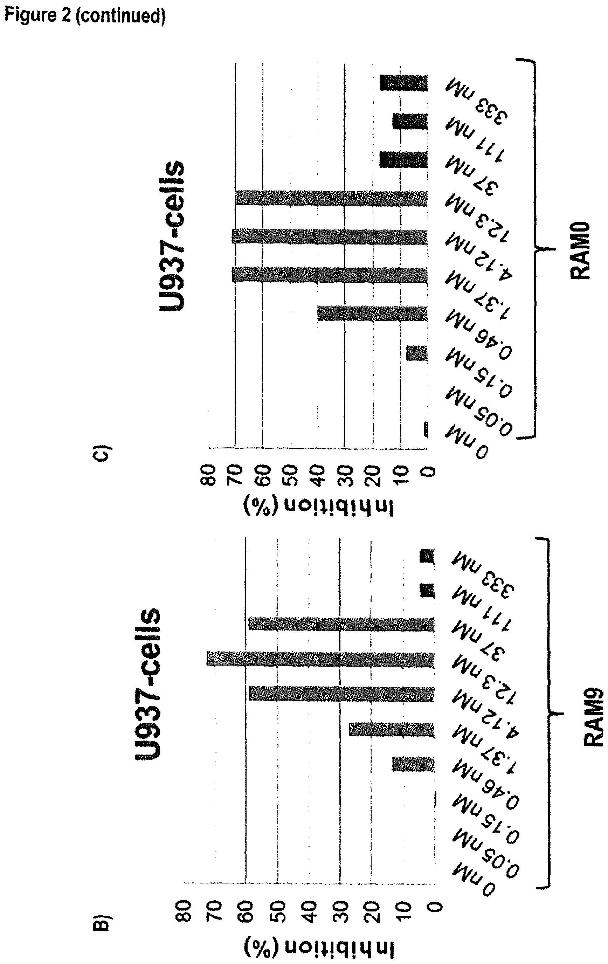

- **Figure 2:**: Inhibition of monocyte migration by anti-MIF antibodies in an *in vitro* cell migration assay. Dose response curves were recorded for the RAM9 and RAM0 antibodies using the human monocytic cell lines THP-1 (Figure 2A) and U937 (Figure 2B, 2C) as indicated in Example 2. The antibody concentrations and the percentage of inhibition of migration in the respective cell lines are as indicated in the Figure.

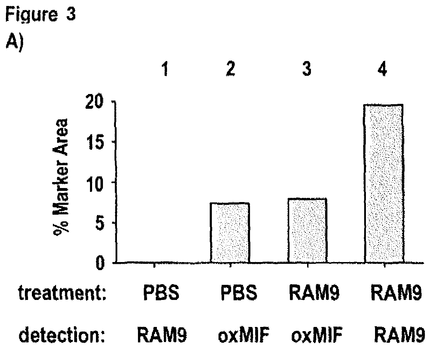

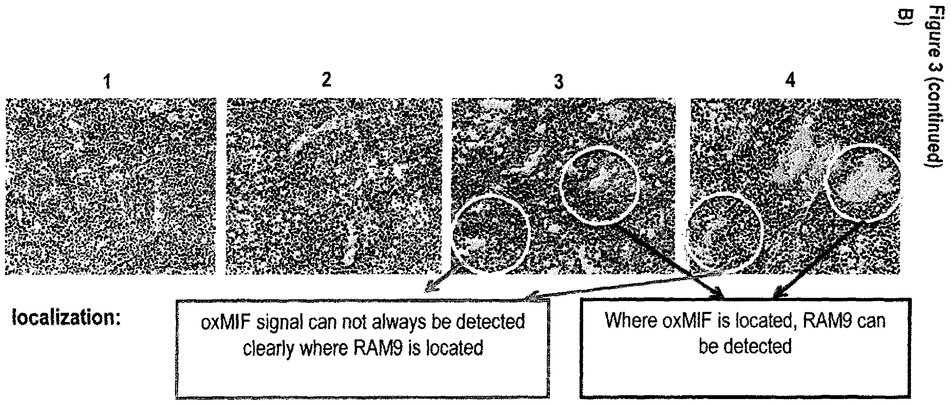

- **Figure 3:**: Tissue distribution of anti-MIF antibodies and oxMIF in a Chronic Lymphocytic Leukemia (CLL) model. A Eµ-myc lymphoma xenograft model in CD1 and C57B16 mice was used to assess RAM9 tissue distribution. The experiment included four treatment groups (each with 6 mice per group): PBS; BaxC3 (60 mg/kg); RAM0 (60 mg/kg); and RAM9 (60 mg/kg). The tumors were allowed to establish for 5 days. The animals were treated every other day for up to 15 days depending on survival time. Tumors (lymph nodes) were resected after the treatment period, snap frozen and embedded for tissue section preparation. Tissue sections were stained for RAM9 tissue penetration and for oxMIF. Full slides were scanned by an Olympus slide scanning microscope (VS120). Digital full slide images were analyzed using Definiens Tissue Studio™ to compare RAM9 and oxMIF distribution (stained area) in the PBS and RAM9 treated groups; and distribution of human IgG in all treatment groups. Tissue images are shown in Figure 3B. A numeric readout is shown in Figure 3A.

### Definitions and General Techniques

Unless otherwise defined herein, scientific and technical terms used in connection with the present invention shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2nd ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990), which are incorporated herein by reference. "MIF" or "macrophage migration inhibitory factor" refers to the protein, which is known as a critical mediator in the immune and inflammatory response, and as a counterregulator of glucocorticoids. MIF includes mammalian MIF, specifically human MIF (Swiss-Prot primary accession number: P14174), wherein the monomeric form is encoded as a 115 amino acid protein but is produced as a 114 amino acid protein due to cleavage of the initial methionine. "MIF" also includes "GIF" (glycosylation-inhibiting factor) and other forms of MIF such as fusion proteins of MIF. The numbering of the amino acids of MIF starts with the N-terminal methionine (amino acid 1) and ends with the C-terminal alanine (amino acid 115).

"oxidized MIF" or oxMIF is defined for the purposes of the invention as an isoform of MIF that occurs by treatment of MIF with mild oxidizing reagents, such as Cystine. As has been shown by the present inventors, recombinant oxMIF that has been treated this way comprises isoform(s) of MIF that share structural rearrangements with oxMIF that (e.g.) occurs *in vivo* after challenge of animals with bacteria.

redMIF is defined for the purposes of this invention as reduced MIF and is MIF which does not bind to RAB0, RAB9 and/or RAB4.

The anti-oxMIF antibodies used in this invention are able to discriminate between ox and red MIF, which are generated by mild oxidation or reduction, respectively. The anti-oxMIF antibodies are useful to specifically detect oxMIF. Discrimination between these conformers is assessed by ELISA or surface plasmon resonance. Both techniques can be performed as well known to a person skilled in the art and as described below.

### Assessing differential binding of the antibodies by surface plasmon resonance.

Binding kinetics of oxMIF and redMIF to antibody RAB9 and RAB0 are examined by surface plasmon resonance analysis, e.g. using a Biacore™ 3000 System. The antibodies were coated on a CM5 (= carboxymethylated dextran) chip and recombinant MIF protein, pre-incubated with 0.2% Proclin®300, were injected. (Proclin®300 consists of oxidative isothiazolones that stabilize the oxMIF structure). In native HBS-EP buffer (= Biacore™ running buffer) without addition of ProClin®300, none of the recombinant MIF proteins bound to RAB9, RAB0 or to the reference antibody (irrelevant isotype control antibody) used as negative (background) binding control.

In a preferred embodiment, oxMIF is MIF which is differentially bound by antibody RAB9, RAB4 and/or RAB0 or an antigen-binding fragment thereof, meaning that these antibodies do bind to oxMIF while redMIF is not bound by either one of these antibodies.

In other embodiments, the anti-oxMIF antibodies, e.g., the antibodies mentioned above or an antigen-binding portion thereof bind oxMIF with a K_{D} of less than 100 nM, preferably a K_{D} of less than 50 nM, even more preferred with a K_{D} of less than 10 nM. Particularly preferred, the antibodies of the invention bind to oxMIF with a K_{D} of less than 5 nM.

The antibodies of the invention, as defined hereinabove and hereinafter, have the same specificities. They thus also show similar results in the experiments as carried out by the present inventors.

(Non-)binding of an antibody, e.g. RAB9, RAB4 or RAB0 or RAM9, RAM4 or RAM0 (to oxMIF or redMIF) can be determined as generally known to a person skilled in the art, examples being any one of the following methods: ELISA with recombinant MIF in its reduced or oxidized state, or surface plasmon resonance using recombinant MIF in its reduced or oxidized state, like the well known Biacore™ assay, described above.

A preferred method for the determination of binding is surface plasmon resonance of an antibody to e.g. rec. (ox)MIF whereupon "binding" is meant to be represented by a K_{D} of less than 100 nM preferably less than 50 nM, even more preferred less than 10 nM whereas the non-binding to redMIF is characterized by a K_{D} of more than 400 nM. "Binding" and "specific binding" is used interchangeably here to denote the above. "Differential binding" in the context of this application means that a compound, in particular the antibodies as described herein, bind to oxMIF (e.g., with the K_{D} values mentioned above) while they do not bind to redMIF (with non-binding again being defined as above).

An "antibody" refers to an intact antibody or an antigen-binding portion that competes with the intact antibody for (specific) binding. See generally, Fundamental Immunology, Ch. 7 (Paul, W., ed., 2nd ed. Raven Press, N.Y. (1989)) (incorporated by reference). The term antibody includes human antibodies, mammalian antibodies, isolated antibodies and genetically engineered forms such as chimeric, camelide/camelized or humanized antibodies, though not being limited thereto.

The term "antigen-binding portion" of an antibody refers to one or more fragments of an antibody that retain the ability to specifically bind to an antigen (e.g., (ox)MIF). Antigen-binding portions may be produced by recombinant DNA techniques or by enzymatic or chemical cleavage of intact antibodies. Antigen-binding portions include e.g. - though not limited thereto - the following: Fab, Fab', F(ab')2, Fv, and complementarity determining region (CDR) fragments, single-chain antibodies (scFv), chimeric antibodies, antibodies and polypeptides that contain at least a portion of an antibody that is sufficient to confer specific antigen binding to the polypeptide, i.e., ox or redMIF. From N-terminus to C-terminus, both the mature light and heavy chain variable domains comprise the regions FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md. (1987 and 1991)), Chothia et al. J. Mol. Biol. 196:901-917 (1987), or Chothia et al., Nature 342:878-883 (1989). An antibody or antigen-binding portion thereof can be derivatized or linked to another functional molecule (e.g., another peptide or protein). For example, an antibody or antigen- binding portion thereof can be functionally linked to one or more other molecular entities, such as another antibody (e.g., a bispecific antibody or a diabody), a detectable agent, a cytotoxic agent, a pharmaceutical agent, and/or a linking molecule.

The term "KD" refers here, in accordance with the general knowledge of a person skilled in the art to the equilibrium dissociation constant of a particular antibody with the respective antigen. This equilibrium dissociation constant measures the affinity. The affinity determines how much complex is formed at equilibrium (steady state where association balances dissociation) (here: ox or redMIF and antibody).
ka= association rate constant [M-1 s-1]
kd =dissociation rate constant [s-1]
KD = equilibrium dissociation constant = kd/ka [M]

The term "human antibody" refers to any antibody in which the variable and constant domains are human sequences. The term encompasses antibodies with sequences derived from human genes, but which have been changed, e.g. to decrease possible immunogenicity, increase affinity, eliminate cysteines that might cause undesirable folding, etc. The term encompasses such antibodies produced recombinantly in non-human cells, which might e.g. impart glycosylation not typical of human cells.

The term "humanized antibody" refers to antibodies comprising human sequences and containing also non-human sequences; in particular, a "humanized antibody" refers to a non-human antibody where human sequences have been added and/or replace the non-human sequences.

The term "camelized antibody" refers to antibodies wherein the antibody structure or sequences has been changed to more closely resemble antibodies from camels, also designated camelid antibodies. Methods for the design and production of camelized antibodies are part of the general knowledge of a person skilled in the art.

The term "chimeric antibody" refers to an antibody that comprises regions from two or more different species. The term "isolated antibody" or "isolated antigen-binding portion thereof" refers to an antibody or an antigen-binding portion thereof that has been identified and selected from an antibody source such as a phage display library or a B-cell repertoire.

The production of the anti-(ox)MIF antibodies according to the present invention includes any method for the generation of recombinant DNA by genetic engineering, e.g. via reverse transcription of RNA and/or amplification of DNA and cloning into expression vectors. In some embodiments, the vector is a viral vector, wherein additional DNA segments may be ligated into the viral genome. In some embodiments, the vector is capable of autonomous replication in a host cell into which it is introduced (e.g., bacterial vectors having a bacterial origin of replication and episomal mammalian vectors). In other embodiments, the vector (e.g., non-episomal mammalian vectors) can be integrated into the genome of a host cell upon introduction into the host cell, and thereby replicated along with the host genome. Moreover, certain vectors are capable of directing the expression of genes to which they are operatively linked. Such vectors are referred to herein as "recombinant expression vectors" (or simply, "expression vectors").

Anti-(ox)MIF antibodies can be produced *inter alia* by means of conventional expression vectors, such as bacterial vectors (e.g., pBR322 and its derivatives), or eukaryotic vectors. Those sequences that encode the antibody can be provided with regulatory sequences that regulate the replication, expression and/or secretion from the host cell. These regulatory sequences comprise, for instance, promoters (e.g., CMV or SV40) and signal sequences. The expression vectors can also comprise selection and amplification markers, such as the dihydrofolate reductase gene (DHFR), hygromycin-B-phosphotransferase, and thymidine-kinase. The components of the vectors used, such as selection markers, replicons, enhancers, can either be commercially obtained or prepared by means of conventional methods. The vectors can be constructed for the expression in various cell cultures, e.g., in mammalian cells such as CHO, COS, HEK293, NSO, fibroblasts, insect cells, yeast or bacteria such as *E.coli.* In some instances, cells are used that allow for optimal glycosylation of the expressed protein.

The anti-(ox)MIF antibody light chain gene(s) and the anti-(ox)MIF antibody heavy chain gene(s) can be inserted into separate vectors or the genes are inserted into the same expression vector. The antibody genes are inserted into the expression vector by standard methods, e.g., ligation of complementary restriction sites on the antibody gene fragment and vector, or blunt end ligation if no restriction sites are present.

The production of anti-(ox)MIF antibodies or antigen-binding fragments thereof may include any method known in the art for the introduction of recombinant DNA into eukaryotic cells by transfection, e.g. via electroporation or microinjection, For example, the recombinant expression of anti-(ox)MIF antibody can be achieved by introducing an expression plasmid containing the anti-(ox)MIF antibody encoding DNA sequence under the control of one or more regulating sequences such as a strong promoter, into a suitable host cell line, by an appropriate transfection method resulting in cells having the introduced sequences stably integrated into the genome. The lipofection method is an example of a transfection method which may be used according to the present invention.

The production of anti-(ox)MIF antibodies may also include any method known in the art for the cultivation of said transformed cells, e.g. in a continuous or batchwise manner, and the expression of the anti-(ox)MIF antibody, e.g. constitutive or upon induction. It is referred in particular to WO 2009/086920 for further reference for the production of anti-(ox)MIF antibodies. In a preferred embodiment, the anti-(ox)MIF antibodies as produced according to the present invention bind to oxMIF or an epitope thereof. Particularly preferred antibodies in accordance with the present invention are antibodies RAB9, RAB4 and/or RAB0, as well as RAM9, RAM4 and/or RAM0.

The sequences of these antibodies are partly also disclosed in WO 2009/086920; see in addition the sequence list of the present application and the following:
SEQ ID NO: 1 for the amino acid sequence of the light chain of RAB9:
SEQ ID NO: 2 for the amino acid sequence of the light chain of RAB4:
SEQ ID NO: 3 for the amino acid sequence of the light chain of RAB0:
SEQ ID NO: 4 for the amino acid sequence of the light chain of RAB2:
SEQ ID NO: 5 for the amino acid sequence of the heavy chain of RAB9:
SEQ ID NO: 6 for the amino acid sequence of the heavy chain of RAB4:
SEQ ID NO: 7 for the amino acid sequence of the heavy chain of RAB0:
SEQ ID NO: 8 for the amino acid sequence of the heavy chain of RAB2:
SEQ ID NO: 9 for the amino acid sequence of RAM0hc:
SEQ ID NO: 10 for the amino acid sequence of RAM0lc:
SEQ ID NO: 11 for the amino acid sequence of RAM9hc:
SEQ ID NO: 12 for the amino acid sequence of RAM9lc:
SEQ ID NO: 13 for the amino acid sequence of RAM4hc:
SEQ ID NO: 14 for the amino acid sequence of RAM4lc:

The anti-MIF antibody of the invention is preferably an isolated monoclonal antibody. The anti-MIF antibody can be an IgG, an IgM, an IgE, an IgA, or an IgD molecule. In other embodiments, the anti-MIF antibody is an IgG1, IgG2, IgG3 or IgG4 subclass. In other embodiments, the antibody is either subclass IgG1 or IgG4. In other embodiments, the antibody is subclass IgG4. In some embodiments, the IgG4 antibody has a single mutation changing the serine (serine228, according to the Kabat numbering scheme) to proline. Accordingly, the CPSC sub-sequence in the Fc region of IgG4 becomes CPPC, which is a sub-sequence in IgG1 (Angal et al. Mοl Immunol. 1993, 30, 105-108).

Additionally, the production of anti-(ox)MIF antibodies may include any method known in the art for the purification of an antibody, e.g., via anion exchange chromatography or affinity chromatography. In one embodiment the anti-(ox)MIF antibody can be purified from cell culture supernatants by size exclusion chromatography.

The terms "center region" and "C-terminal region" of MIF refer to the region of human MIF comprising amino acids 35-68 and aa 86-115, respectively, preferably aa 50-68 and aa 86 to 102 of human MIF, respectively. Particularly preferred antibodies of the present invention bind to either region aa 50-68 or region aa 86-102 of human MIF. This is also reflected by the preferred antibodies of the invention, like RAB0, RAB4 RAB2 and RAB9 as well as RAM4, RAM9 and RAM0 which bind as follows:

| | |
|---|---|
| RAB4 and RAM4: | aa 86-102 |
| RAB9 and RAM9: | aa 50-68 |
| RAB0 and RAM0: | aa 86-102 |
| RAB2: | aa 86 - 102 |

The term "epitope" includes any protein determinant capable of specific binding to an immunoglobulin or an antibody fragment. Epitopic determinants usually consist of chemically active surface groupings of molecules such as exposed amino acids, amino sugars, or other carbohydrate side chains and usually have specific three-dimensional structural characteristics, as well as specific charge characteristics.

The term "vector" refers to a nucleic acid molecule capable of transporting another nucleic acid to which it has been linked. In some embodiments, the vector is a plasmid, i.e., a circular double stranded DNA loop into which additional DNA segments may be ligated.

The term "host cell" refers to a cell line, which is able to produce a recombinant protein after introducing an expression vector. The term "recombinant cell line", refers to a cell line into which a recombinant expression vector has been introduced. It should be understood that "recombinant cell line" means not only the particular subject cell line but also the progeny of such a cell line. Because certain modifications may occur in succeeding generations due to either mutation or environmental influences, such progeny may not, in fact, be identical to the parent cell, but are still included within the scope of the term "recombinant cell line" as used herein.

The host cell type according to the present invention is e.g. a COS cell, CHO cell or, e.g., an HEK293 cell, or any other host cell known to a person skilled in the art, thus also for example including bacterial cells, like e.g. *E.coli* cells. In one embodiment, the anti-MIF antibody is expressed in a DHFR-deficient CHO cell line, e.g., DXB11, and with the addition of G418 as a selection marker. When recombinant expression vectors encoding antibody genes are introduced into CHO host cells, the antibodies are produced by culturing the host cells for a period of time sufficient to allow for expression of the antibody in the host cells or secretion of the antibody into the culture medium in which the host cells are grown.

Anti-(ox)MIF antibodies can be recovered from the culture medium using standard protein purification methods.

An optional second active ingredient of a combination therapy as encompassed by the present invention is a chemotherapeutic.

Chemotherapeutic agents in the general sense thereof, are compounds, which can be used for the treatment of a disease or disorder that arises from bacterial, viral or parasitic infection or that is due to transformation of normal cells (cancer). One particular indication of chemotherapy is cancer. Chemotherapeutic agents can act for example by killing cells that divide more rapidly than other cells, and thus target cancer cells which commonly divide more rapidly than non-cancerous cells, Most chemotherapeutic agents work by impairing cell division at one of several stages of the cell cycle. Thus, they are able to target those cells that divide more rapidly. Chemotherapeutic agents can be either cytostatic, i.e., they slow down or abrogate the growth or division of cells; other chemotherapeutic agents can cause damage to cells and kill them; in that case they are termed cytotoxic. Most cytotoxic drugs inflict a damage that *per se* does not suffice to kill a cell but that generates a stimulus to initiate programmed cell death (apoptosis).

In general, major classes of chemotherapeutic drugs are alkylating agents, anti-metabolites, anthracyclines, plant alkaloids, topoisomerase inhibitors and other anti-tumour agents. Most commonly, as mentioned above, these drugs affect one or several stages of the cell cycle; they can also affect DNA synthesis or DNA integrity. Other chemotherapeutics do not directly interfere with DNA. These are newer classes of chemotherapeutics and can include monoclonal antibodies and tyrosine kinase inhibitors like imatinib mesylate. Other examples are chemotherapeutic hormones and hormone antagonists, e.g. glucocorticosteroids.

Examples for alkylating agents, which alkylate nucleophilic functional groups are mechlorethamine, cyclophosphamide, chlorambucil, melphalane, trofosfamide, ifosfamide, carmustine, lomustine, dacarbazine, temozolomide, mitomycine C and many others. Cisplatin, carboplatin, oxaliplatin and other platinum containing compounds form stable complexes with DNA.

Cytotoxic anti-metabolites are folic acid analogues (e.g., methotrexate/aminopterin, raltitrexed, pemetrexed), purine analogs (e.g., 6-mercaptopurine, azathioprine, thioguanine, fludarabine, cladribine) or pyrimidine analogs (cytarabine, gemcitabine, deazacytidine, 5-fluorouracil and its prodrugs including capecitabine). Antimetabolites either inhibit DNA-synthesis by interfering with crucial steps in the *de novo* synthesis of purine and pyrimidine nucleotides or they become incorporated into DNA during the S-phase of the cell cycle, where they interfere with DNA-folding, DNA-repair or methylation. Alternatively, some compounds also become incorporated into RNA.

Examples for alkaloids and terpenoids which are derived from plants and block cell division by preventing microtubule function are vinca-alkaloids and taxanes. Particularly well known vinca-alkaloids are vincristine, vinblastine, vinorelbine and vindesine. Podophyllotoxin is an additional example of a plant-derived compound. An example for a taxane is docetaxel or paclitaxel. Another example is abraxane, an albumin bound paclitaxel. Estramustin is an example of a synthetic compound that targets tubulin.

Examples of topoisomerase inhibitors, which are inhibitors of enzymes that maintain the topology of DNA, include camphtotecines like irinotecan and topotecan (type 1 topoisomerase inhibitors) or amsacrin, etoposide, etoposide phosphate and teniposide (topoisomerase-type 2 inhibitors).

Finally, examples of antineoplastic intercalating agents include dactinomycin, doxorubicin, epirubicin, bleomycin and others.

A comprehensive overview is comprised in Goodman and Gilman, The Pharmacological Basis of Therapeutics, 12th Edition, "General Principles of Cancer Chemotherapy".

The following are examples for alkylating agents:
Mechlorethamine
Cyclophosphamide Ifosfamide
Melphalan
Chlorambucil
Procarbazine (N-methylhydrazine, MIH)
Busulfan
Camustine (BCNU)
Streptozocin
(streptozotocin)
Bendamustine
Dacarbazine (DTIC; dimethyltriazenol midazole carboxamide)
Temozolomide
Cisplatin, carboplatin, oxaliplatin
Antimetabolites are exemplary represented by
Methotrexate (Amethopterin)
Pemetrexed
Fluorouracil (5-fluorouracil; 5-FU), capecitabine
Cytarabine (cytosine arabinoside)
Gemcitabine
5-aza-cytidine
Deoxy-5-aza-cytidine
Mercaptopurine (6-mercaptopurine; 6-MP)
Pentostatin (2'-deoxycoformycin)
Fludarabine
Clofarabine
Nelarabine
while Natural Products can be selected from:
Vinblastine
Vinorelbine
Vincristine
Paclitaxel, docetaxel
Etoposide
Teniposide
Topotecan
Irinotecan
Dactinomycin
(actinomycin D)
Daunorubicin
(daunomycin, rubidomycin)
Doxorubicin
Yondelis
Mitoxantrone
Bleomycin
Mitomycin C
L-Asparaginase

Examples for Hormones and Antagonists are:
Mitotane (o.*p* DDD)
Prednisone
Hydroxyprogesterone caproate,
medroxyprogesterone acetate, megestrol acetate
Dietyhlstilbestrol, ethinyl estradiol
Tamoxifen, toremifene
Anastrozole, letrozole, exemestane
Testosterone propionate, fluoxymesterone
Flutamide, casodex
Leuprolide
while examples for further agents are:
Hydroxyurea
Tretinoin, arsenic trioxide
Histone deacetylase inhibitor (vorinostat)
Imatinib
Dasatinib, nilotinib
Gefitinib, erlotinib
Sorafenib
Sunitinib
Lapatinib
Bortezomib
Interferon-alfa,
Interleukin-2
Thalidomide
Lenalidomide
Temsirolimus,
Everolimus

Chemotherapeutics have been shown to be successful in alleviation and treatment of cancer. However, most chemotherapeutics are associated with a range of side effects which are in some cases extreme, to the extent that the treatment has to be abrogated. In any case, the side effects place a further burden on the physical and mental health of a patient and should thus be avoided as far as possible. By combining a chemotherapeutic with an anti-MIF antibody administered by using a dosage regimen according to the present invention, it is possible to reduce the amount of the chemotherapeutic agent which is necessary for a given treatment compared to a situation where the chemotherapeutic agent is given as the sole active ingredient. A further possibility is to maintain the dose of the chemotherapeutic as compared to the chemotherapeutic given alone and have a much higher treatment response in the patient.

This increase of the treatment response in the patient also indicates the possibility to achieve a treatment response as with a chemotherapeutic alone, with a combination of anti-MIF antibody administered by using a dosage regimen according to the present invention with a lower dose of chemotherapeutic agent, e.g. in cases where the side effects of the chemotherapeutic do not allow continuous treatment with the higher dose. It was shown by the present inventors that the effect obtained by combining a chemotherapeutic with an anti-MIF antibody showed a much higher treatment response than with either the anti-MIF antibody or the chemotherapeutic agent alone.

A treatment response can easily be determined by a person skilled in the art and refers to diminishing or ameliorating or alleviating a given condition. Methods to determine such a treatment response are well known and can be for example determination of the likelihood or length of survival of a subject having a disease and being treated with a combination of MIF antagonist and chemotherapeutic agent with the likelihood or length of survival in other subjects having the same disease and being treated with either agent alone, or by determining the change of symptoms within one and the same patient over a period of time. An example well known to a person skilled in the art is the Kaplan-Meier-Plot.

Other methods/assays are well known and can be derived for example from general textbooks, like The Pharmacological Basis of Therapeutics, 12th Edition, "General Principles of Cancer Chemotherapy. Introduction", This reference is incorporated hereby in its entirety by reference; additional methods/assays are those as described in the present examples.

Preferred chemotherapeutics that are encompassed by the uses of the present invention are 5-fluorouracil, leucovorin, doxorubicin and/or gemcitabine. 5-fluorouracil can be used in a preferred embodiment in combination with leucovorin. Doxorubicin can be used in a preferred embodiment in combination with cisplatin. Also preferred is e.g. abraxane.

If two or more chemotherapeutics are used together with the anti-MIF antibodies in accordance with the invention, the timing and route of administration of the two or more chemotherapeutics may or may not be the same with respect to each other. For instance, these two or more chemotherapeutics can be administered simultaneously with regard to each other, or they can be administered sequentially. For example, if a combination of 5-fluorouracil with leucovorin is used together with an anti-MIF antibody of the invention such as RAM9, leucovorin can be administered intravenously once every two weeks at a dosage of 400 mg/m² body surface area over two hours followed by intravenous administration of 5-fluorouracil at a dosage of 2400 mg/m² body surface area over 46 hours.

Another optional active ingredient of a combination therapy as encompassed by the present invention is an antibody specific to the human epidermal growth factor receptor (known synonyms of this receptor are EGF receptor, EGFR, ErbB-1 and HER1). A preferred antibody specific to the human epidermal growth factor receptor is panitumumab. In accordance with the present invention, antibodies to the human epidermal growth factor receptor including panitumumab are used at the typical dosages for such anti-EGFR antibodies, which are well known to the person skilled in the art. In accordance with the present invention, a combination therapy with an antibody specific to the human epidermal growth factor receptor may optionally be used in combination with further optional active ingredients of the invention such as chemotherapeutics. Antibodies to the human epidermal growth factor receptor including panitumumab are preferably administered intravenously. In a preferred embodiment of the above combinations the anti-MIF antibody is selected from the group of RAM9, RAM4 and RAM0.

"Cancer" in the present context encompasses all disorders or diseases in which a cell or a group of cells displays uncontrolled growth, invasion (intrusion and destruction of adjacent tissues) and sometimes metastasis. The cancer to be treated according to the present invention is a solid tumor. As used herein, the term "solid tumor" includes the primary solid tumor. If (one or more) solid tumor metastases are present, the term "solid tumor" also includes these solid tumor metastases. Further, in a preferred embodiment, the cancer can be oxMIF-related. oxMIF-related cancers are e.g. lymphoma, sarcoma, prostate cancer, colorectal cancer (also known as CRC) and colon cancer, bladder cancer, pancreas cancer, ovarian cancer, melanoma, hepatocellular carcinoma, ovarian cancer, lung cancer, breast cancer and pancreatic cancer, as well as endometriosis. More preferably, the cancer is ovarian cancer, non-small cell lung cancer or colorectal cancer. In another preferred embodiment, the cancer can be a lymphoma, sarcoma, prostate cancer, colorectal cancer (also known as CRC) and colon cancer, bladder cancer, pancreas cancer, ovarian cancer, melanoma, hepatocellular carcinoma, ovarian cancer, lung cancer, breast cancer and pancreatic cancer, as well as endometriosis, More preferably, the cancer is ovarian cancer, non-small cell lung cancer or colorectal cancer.

The anti-MIF antibody as used according to the invention is to be administered intravenously. The administration of the chemotherapeutic can principally be by all known routes.

Further possible dosage forms for the chemotherapeutic which are also envisaged by the present application are dosage forms for oral administration such as tablets, capsules, sachets or pills. The granules can be used as such as a preferred dosage form, can be filled into capsules or sachets or can be further compressed into tablets or pills.

Further dosage forms for the chemotherapeutic which are also encompassed by the present application are drinks or syrups, elixirs, tinctures, suspensions, solutions, hydrogels, films, lozenges, chewing gums, orally disintegrating tablets, mouth-washes, toothpaste, lip balms, medicated shampoos, nanosphere suspensions and microsphere tablets, as well as aerosols, inhalers, nebulisers, smoking or freebase powder forms and dosage forms for topical application like creams, gels, liniments or balms, lotions, ointments, ear drops, eye drops and skin patches.

Further encompassed are suppositories for the chemotherapeutic which can be used e.g. rectally or vaginally. All these dosage forms are well-known to a person skilled in the art.

Dosage forms for the chemotherapeutic in accordance with the present invention are oral forms like granules, coated granules, tablets, enteric coated tablets, pellets, suppositories and emulsions. Even more preferred are granules and tablets. Other dosage forms are topical dosage forms. A preferred administration route for the chemotherapeutic agent is oral application (e.g., a granule, liquid, sachet or tablet). A further preferred application form for the chemotherapeutic is topical application, wherein a topical application can encompass an application to the skin and/or a spray, like a nasal spray or inhaler. A further preferred administration route for a chemotherapeutic is an intravenous application or an application via a subcutaneous injection (including slow release formulations).

The term "combination" or "combination therapy" are used interchangeably here. They refer to a dosing regimen where the anti-MIF antibody is administered together with or sequentially to the chemotherapeutic or *vice versa.* The dosing regimen would be typically daily for chemotherapeutics and every 7 days for the anti-MIF antibody.

The term "half-life" as referred to herein refers to the time that a substance (e.g. an anti-MIF antibody as used according to the invention, or a chemotherapeutic as optionally also used according to the invention) needs in order to lose half of its biological activity in the respective subject (e.g. in rodents such as mice and primates such as monkeys or humans). The half-life can for instance be measured by determining plasma concentrations of the respective substance in the subject by appropriate assays known in the art or described herein. Some substances, which in particular include anti-MIF antibodies, exhibit a biphasic elimination kinetics with an initial fast elimination phase for the substance and a subsequent slower elimination phase for the substance. For such substances such as anti-MIF antibodies, an initial (shorter) half-life and a subsequent terminal (longer) half-life can be determined. It was found that the terminal half-life of anti-MIF antibodies in primates is much longer than their terminal half-life in mice (about 5 to 10 days as compared to about 1.5 days in mice).

Preferred dosing regimens for the anti-MIF antibodies, either alone or in combination with a chemotherapeutic, are:
Generally, the anti-MIF antibody is to be administered intravenously to the human subject at a dosage in the range of 1 mg per kg body weight to 10 mg per kg body weight, For example, the anti-MIF antibody can be administered at a dosage of 1 mg per kg body weight, 2 mg per kg body weight, 3 mg per kg body weight, 4 mg per kg body weight, 5 mg per kg body weight, 6 mg per kg body weight, 7 mg per kg body weight, 8 mg per kg body weight, 9 mg per kg body weight, or 10 mg per kg body weight. In a preferred embodiment, the anti-MIF antibody is to be administered at a dosage in the range of 2.5 mg per kg body weight to 8 mg per kg body weight. In a further preferred embodiment, the anti-MIF antibody is to be administered at a dosage in the range of 5.5 mg per kg body weight to 6.5 mg per kg body weight, preferably at a dosage of 6 mg per kg body weight. In this embodiment the cancer is preferably pancreatic cancer or ovarian cancer. In a further preferred embodiment, the anti-MIF antibody is to be administered at a dosage in the range of 2.5 mg per kg body weight to 8 mg per kg body weight, preferably at a dosage in the range of 3 mg per kg body weight to 4 mg per kg body weight, and the cancer is prostate cancer. In a further preferred embodiment, the anti-MIF antibody is to be administered at a dosage of 10 mg per kg body weight. In a further preferred embodiment, the anti-MIF antibody is to be administered at a dosage in the range of 4.5 mg per kg body weight to 5.5 mg per kg body weight, preferably at a dosage of 5 mg per kg body weight. In a very preferred embodiment, the anti-MIF antibody is to be administered at a dosage of 10 mg per kg body weight and the cancer is selected from the following group: colorectal cancer, non-small cell lung cancer and ovarian cancer. In a further very preferred embodiment, the anti-MIF antibody is to be administered at a dosage of 5 mg per kg body weight and the cancer is selected from the following group: colorectal cancer, non-small cell lung cancer and ovarian cancer.

The above dosages can be used when using anti-MIF antibodies alone, and they can also be used when using anti-MIF antibodies in combination with chemotherapeutic agent(s). Furthermore, unless indicated otherwise, the above dosages are applicable to all cancers that can be treated according to the present invention.

It is to be understood that each dosage of the present invention disclosed herein refers to the respective specific dosage but also includes a respective dosage range of +/-10 %.

For each respective anti-MIF antibody dosage according to the invention, administration of anti-MIF antibodies may include a loading dose. That is, during an initial period of administration, a loading dose of the anti-MIF antibody, which is higher than the respective anti-MIF antibody dosage, may be administered. Such a loading dose according to the invention may be a single dose of an anti-MIF antibody, but a loading dose is not limited to a single dose. Alternatively, the invention may also be carried out without administration of a loading dose.

As explained above, it is possible to administer the anti-MIF antibody together with the optional chemotherapeutic agent(s) or sequentially. "Together with" in this context means that not more than 10 minutes have passed between the administration of the anti-MIF antibody and the administration of the chemotherapeutic. "Sequentially" means that more than 10 minutes have passed between the administration of the anti-MIF antibody and the administration of the chemotherapeutic agent. The time period can then be more than 10 min., more than 30 minutes, more than 1 hour, more than 3 hours, more than 6 hours or more than 12 hours.

Anti-MIF antibody and the optional chemotherapeutic agents are principally dosed in a way to ensure that the respective compounds are present within the body during the same time period (for a certain time span). An anti-MIF antibody has a terminal half-life of typically 5 to 10 days in primates, chemotherapeutic agents a half-life of 2-48 hours.

Therefore, the above combination therapy also explicitly encompasses a sequential dosing regime where the skilled person takes into account the well-known half-life of the respective chemotherapeutic drug in question and the antibody in question.

In view of the fact that the antibodies according to the invention have a terminal half-life of typically 5 to 10 days in primates, administration of the anti-MIF antibody in question to humans according to the invention is preferably only every 5 days, every 6 days, every 7 days, every 8 days, every 9 days, or every 10 days. In a very preferred embodiment, the anti-MIF antibody is to be administered to humans every 7 days. These intervals of administration can be used for any of the dosages defined above (including any of the dosages in the range of 1 mg per kg body weight to 10 mg per kg body weight as defined above). The optional chemotherapeutic drug to be administered in a combination therapy with such an antibody encompassed by the present invention has in a typical embodiment a half-life of 2 - 48 h; therefore, administration of the chemotherapeutic could be every 5 hours, every 6 hours, three times a day, twice a day, once daily, once a week or once per three week cycle in a typical embodiment.

Dosing of chemotherapeutics agent, as well as the combined dosing with antibodies, in accordance with the present invention, however, will need to be determined by the practitioner on a case-by-case basis according to the specific disorder to be treated and the particulars of the afflicted subject. The person of skill in the art is aware of the respective guidelines for a given chemotherapeutic agent.

As a general understanding in curative chemotherapy, one would wish to apply the highest tolerated dose of the chemotherapeutic to achieve the desired dose intensity. The dose is reduced only if there is toxicity (i.e., neutrophil counts < 4000 (but >2500) = administer half the dose, see cisplatin or cyclophosphamide). Most chemotherapeutic agents are administered on the basis of (m)g/m² body surface. Differences in tolerance and efficacy between mouse, rat and man are typically accounted for by basing the dose on body surface.

In a particularly preferred embodiment, the active ingredient would be an ingredient which should be delivered with a controlled, e.g. a delayed release. That is, the orally administrable dosage forms of the present invention comprising such an active ingredient might be provided with a coating. Thus, in a preferred embodiment the present invention is directed to granules with coatings and in particular to granules comprising active ingredients which shall be released in a controlled manner, whereby these granules have a coating.

More preferred, this coating is pharmacologically acceptable coating and particularly preferred is an enteric coating, a prolonged release coating or a delayed release coating; all such coatings are well known to a person skilled in the art.

A subset of *in vivo* protective anti-MIF mAbs (e.g. RAB9, RAB4, RABO), are directed against the pro-inflammatory cytokine MIF (Macrophage Migration Inhibitory Factor) were shown to be highly selective for a redox dependent MIF isoform.

A particularly preferred antibody is antibody RAB9.

Another particularly preferred antibody is antibody RAM4.

Yet another particularly preferred antibody is antibody RAM0.

A very preferred antibody is antibody RAM9.

Generally, administration of the anti-MIF antibodies at the dosages according to the invention is advantageous in that it results in a high efficacy of the cancer treatment. The combination therapy of the anti-MIF antibody with the optional chemotherapeutic has additional advantages in that it results in a synergistic effect of both components.

The present invention will be in the following described by way of the examples, whereby the examples shall be considered by no means as limiting the present invention.

### REFERENCE EXAMPLES

### A) GCO-assay for antibody screening:

A THP1 suspension culture is centrifuged and cells are resuspended in fresh full medium to a cell density of 10⁶ cells per ml. This culture is transferred into wells of a 96-well microplate (90 µl/well) and a potential anti-MIF antibody is added to give a final concentration of 75 µg/ml. Each antibody is tested in triplicate. After o/n incubation at 37°C dexamethasone is added to give a concentration of 2 nM and after one hour incubation at 37°C LPS is added (3 ng/ml final concentration). After further six hours incubation at 37°C the supernatant is harvested and the IL-6 concentrations are determined in a commercially available ELISA. The results of the triplicates are averaged and the percentage of IL-6 secretion is determined in comparison to the control antibodies. Antibodies that result in an IL-6 secretion of less than 75% are evaluated as positive.

### B) Assay for determination of IC₅₀ values

The experimental procedure is carried out as described for the screening assay with the exception that increasing amounts of antibody are used (typically from 1 - 125 nM). The resultant dose response curve is expressed as % inhibition in comparison to a negative control antibody. This curve is used for calculation of the maximum inhibitory effect of the antibody (%Inh max) and the antibody concentration that shows 50% of the maximum inhibitory effect (IC₅₀).

### C) Inhibition of cell proliferation

Serum stimulates secretion of MIF in quiescent NIH/3T3 and MIF in turn stimulates cell proliferation. Antibodies inhibiting this endogenous MIF, therefore, decrease the proliferation of quiescent NIH/3T3 cells. The reduction of proliferation is determined by the incorporation of ³H-thymidine.

1000 NIH/3T3 cells per well are incubated in a 96 well plate over the weekend at 37°C in medium containing 10% serum. Cells are then starved over night at 37°C by incubation in medium containing 0.5% serum. The 0.5% medium is removed and replaced by fresh medium containing 10% serum, 75 µg/ml antibody and 5µ Ci/ml of 3H-thymidine, After 16 hours incubation in a CO₂ incubator at 37°C cells are washed twice with 150 µl of cold PBS per well. Using a multi-channel pipette 150 µl of a 5% (w/v) TCA solution per well are added and incubated for 30 minutes at 4°C. Plates are washed with 150 µl PBS. Per well 75 µl of a 0.5M NaOH solution with 0.5% SDS are added, mixed and stored at room temperature. Samples are measured in a β-counter by mixing 5 ml of Ultima Gold (Packard) and 75 µl sample solution. Each determination is done in triplicate and the values are compared with the values of the control antibody by a t-test. Antibodies that significantly reduce proliferation (P<0.05) are evaluated as positive.

### D) Binding studies: Epitope determination of anti-MIF antibodies

Each peptide is diluted in coupling buffer to give a peptide concentration of typically 1 µg/ml added to microplates (NUNC Immobilizer™ Amino Plate F96 Clear) and incubated over night at 4°C (100 µl/well). As controls recombinant full length MIF and PBS are used, The plate is washed 3 times with 200 µl PBST and antibodies (2-4 µg/ml in PBS) are added (100 µl/well) and incubated for 2 hours at room temperature with gentle shaking. The plate is washed 3 times with 200 µl PBST and detection antibody (e.g. Fc specific antihuman IgG/HRP labelled, Sigma) is added (100 µl/well). After incubation for 1 hour at room temperature with gentle shaking, the plate is washed 3 times with 200 µl PBST. Each well is incubated with 100 µl TMB (3,3',5,5'-tetramethylbenzidine) solution (T-0440, Sigma) for 30 minutes in the dark. Staining reaction is stopped by adding 100 µl of 1.8 M H₂SO₄-solution per well. Samples are measured at 450 nm. E) Affinity determination of Fab fragments of anti-MIF antibodies by Biacore™

Typically, 40 RU units of human recombinant MIF are immobilized on a sensor chip with a CM5 (= carboxymethylated dextran) matrix (Biacore™). Fab fragments are injected at a concentration range of typically 6 - 100 nM diluted in HBS-EP. After each cycle the chip is regenerated with 50 mM NaOH + 1 M NaCl. Affinities are calculated according to the 1:1 Langmuir model.

### EXAMPLES

### Example 1: Determination of Effective Dosages for anti-MIF Antibodies in Mouse Cancer Models

Effective dosage ranges for anti-MIF antibodies were tested in various mouse cancer models, as reflected in table 1. The results of the dosage testing including the effective dosages are shown in table 1.

**Table 1:**

| **Disease** | **Model** | **Effective dosage** | **Dosage range tested** |
|---|---|---|---|
| Ovarian Cancer | Xenograft model (IGROV human cancer cell line) | 60 mg/kg | 60 mg/kg q2d |
| | Xenograft model (A2780 human cancer cell line) | 15 mg/kg | 15 mg/kg q2d |
| Prostate Cancer | Xenograft model (PC3 human cancer cell line) | 15-100 mg/kg | 5 - 200 mg/kg (U-shaped) q2d |

"As used herein in general, and as used in the above table and in the following Examples, the term "q2d" refers to a treatment with the anti-MIF antibody every other day.

Surprisingly, it was found that higher dosages of anti-MIF antibodies, which were above the effective dosages indicated in table 1, were less effective than said effective dosages. Thus, the dosage response curves for the anti-MIF antibodies in cancer unexpectedly exhibited a non-monotonic dosage-response curve.

More particularly, the dosage response study for the prostate cancer xenograft model, which is summarized in table 1, last line, above, was carried out as follows, using the RAM9 antibody: PC-3 cells were injected into the flank of MF1 nude mice using matrigel. The study was conducted using a preventive model. Treatment with RAM9 or control antibody was started the day after subcutaneous inoculation of tumor cells into MF1 mice. Antibodies were injected intraperitoneally every other day until animals were killed by cervical dislocation after 20 to 30 days, the time point being determined by the largest (calculated) tumor volume (∼1.5 mL) or the visible suffering of the animals in the control group,

The results are shown in Figure 1. When the dosage of the anti-MIF antibody RAM9 was increased in a stepwise manner from 5 mg/kg q2d to 40 mg/kg q2d, the tumor volume first showed a decrease, corresponding to an increase in treatment efficacy. Surprisingly, however, it was found that when the dosage of the antibody was further increased in a stepwise manner to 100 mg/kg q2d and 200 mg/kg q2d, respectively, the tumor volume did not continue to decrease but increased instead, corresponding to a decrease in treatment efficacy.

Thus, the inventors have found that unexpectedly, the most effective treatment of prostate cancer with an anti-MIF antibody in mice can be carried out at a lower dosage in the range of 15 q2d to 100 mg/kg q2d rather than at a high dosage of the anti-MIF antibody.

In view of the above, effective dosages of anti-MIF antibodies for the treatment of cancer in mice are dosages in the range of 15 to 100 mg/kg q2d, More particularly, the most effective dosages in mice are in the range of around 40-60 mg/kg q2d, i.e. in the range of 25 to 80 mg/kg q2d. These preferred dosages in mice apply to all anti-MIF antibodies, and in particular to the antibodies specifically referred to herein, including the RAM9 antibody, It will be understood that based on these doses and dosage regimes and the half-life of anti-MIF antibodies in mice as determined in Example 4 below, the skilled person will be able to calculate other suitable dosage regimens.

### Example 2: Inhibition of Monocyte Migration by anti-MIF Antibodies

It was next investigated whether such non-monotonic dose-response curves would also be obtained in *in vitro* assays: In particular, inhibition of monocyte migration was assessed by testing the RAM9 and RAM0 antibodies in an *in vitro* monocyte migration assay. The in vitro monocyte migration assay was performed as described in Example 1 of WO 2014/086916. In brief, serum starved human monocytic cells were seeded into porous (5 µm) transwell inserts and cell migration towards different concentrations of anti-oxMIF antibodies (added to the lower transwell chamber) was measured. IC50 was then calculated by plotting % migration inhibition against the concentration of Anti-oxMIF antibody.

The results are shown in Figure 2. As can be seen from the Figure, dosage response curves in human THP-1 and U937 cells showed an optimal inhibition of chemokinesis at anti-MIF antibody concentrations of about 10 nM. Concentrations of 30 nM and above, however, showed a reduced inhibition of chemokinesis. Thus, the *in vitro* migration assay confirmed the results obtained *in vivo,* and in particular the results that
- the dosage-response curve of the anti-MIF antibodies is non-monotonic;
- that a higher dosage of the anti-MIF antibodies results in reduced efficacy; and
- that an optimum lower dosage of the anti-MIF antibodies exists.

### Example 3: Comparison of the Effects of anti-MIF antibodies in Primates and in Rodents

Next, the inventors set out to compare the effects of anti-MIF antibodies in primates and in rodents. First, the affinity of the anti-MIF antibody was tested using a Biacore™ assay: RAM9 antibodies were immobilized to the surface of a Biacore™ CM5 optical sensor chip (GE Healthcare) using standard amine coupling conditions. MIF recombinant proteins from the different species (human, rat and mouse) were diluted in HBS-EP buffer (GE Healthcare) in the presence or not of 0.2% Proclin®300. Concentrations of 40, 80, 160, 320 and 480 nM of the different MIF proteins were applied to the immobilized RAM9, and measurements were done on a Biacore™ 3000 Instrument. Kinetic analysis of the concentration series was performed by local simultaneous association / dissociation fitting of each single binding curve to the iterative Langmuir 1:1 interaction model with mass transfer compensation provided by the BiaEvaluation software.

The results for the binding of the anti-MIF antibody to human and rat MIF are shown in Table 2 below. As can be seen from Table 2, the affinity of the anti-MIF antibody for human MIF was about 3-fold higher than for mouse and rat MIF.

Additionally, the sensitivity of human cell lines to an anti-MIF antibody was compared to a rat cell line in an *in vitro* potency assay which measures the inhibition of cell migration by anti-MIF: The capacity of RAM9 to inhibit random migration of monocytes was tested in a Transwell® Chemokinesis assay (comparable to a Boyden chamber assay). Briefly, cells (human cell line U937 or NR8383 rat macrophages) were seeded into porous Transwell® inserts and cell migration towards different concentrations of RAM9 was measured using a Cellavista device (Innovatis AG, Roche, Basel). IC₅₀ was determined by nonlinear regression equation (4-parameter logistic) of the number of migrated cells against the concentrations of RAM9 (logarithmic scale), following the equation Y= (Ymax-Ymin)/(1+(X/IC₅₀)Expₛₗₒₚₑ + Ymin.

The results are shown in Table 2. As can be seen from Table 2, it was surprisingly found that the human cell lines exhibited an about 10-fold higher sensitivity to the tested anti-MIF antibody as compared to the rat cell line. From these unexpected findings, it was concluded that anti-MIF antibodies are about 10-fold more potent in human *in vitro* assays than in rodent *in vitro* assays.

**Table 2:**

| | **Primates** | **Rodents** | |
|---|---|---|---|
| | **Human** | **Rat** | **Mouse** |
| Biacore™ (Affinity) | 1.7 ± 0.3 nM | 5.6 ± 1.1 nM | 6.9 ± 1.5nM |
| Potency (IC 50) | 1 ± 1.1 nM | 11 ± 5 nM | |

### Example 4: Pharmacokinetic Parameters of anti-MIF Antibodies in Mice and Cynomolgus Monkeys

In order to find appropriate dosing intervals of anti-MIF antibodies *in vivo,* the pharmacokinetic parameters of the RAM9 antibody in mice were determined, Nude mice were injected intravenously with two concentrations of RAM9 (50 and 100 mg/kg). The antibody was well tolerated and a rapid initial decline of the antibody plasma concentration was observed, while at later time points the decrease was slower. Accordingly, the data were adequately described by assuming a biexponential decay, i.e. a rapid decline in the central compartment and a more protracted elimination from a peripheral compartment. Resulting antibody concentration curves were therefore subjected to non-linear least squares curve fitting to an equation describing a biexponential (two compartment model) decay using a Marquardt-Levenberg algorithm. The results are shown in Table 3.

**Table 3:**

| **Species** | **Mice** | | **Cynomolgus monkey** | | |
|---|---|---|---|---|---|
| Dosage (mg/kg) | 50 | 100 | 25 | 100 | 250 |
| Initial half-life (h) | 4.6 | 6.1 | 38.7 | 42.9 | 11.9 |
| Terminal half-life (h) | 35.3 | 35.3 | 231 | 196 | 118 |
| Cmax (µg/mL) | 585 | 1100 | 487 | 1766 | 4021 |

As can be seen from Table 3, the RAM9 antibody exhibited a short initial half-life of about 5 hours and a short terminal half-life of 35.3 hours at the dosages tested in mice. These data in mice suggested that the pharmacokinetic properties of the antibody would not be favorable for an administration of anti-MIF antibodies at dosing intervals of more than two days.

Despite these results, the inventors, however, also determined pharmacokinetic parameters in cynomolgus monkeys. The results are also shown in Table 3. It was found that in contrast to the data obtained in mice, the RAM9 antibody exhibited a much longer initial half-life and also a much longer terminal half-life of 5 to 10 days in cynomolgus monkeys at all dosages tested. Thus, it was concluded that as opposed to the results obtained in mice, anti-MIF antibodies exhibit much more favorable pharmacokinetic properties in primates. The pharmacokinetic properties of the anti-MIF antibodies are therefore compatible with an administration of the anti-MIF antibodies to primates including humans at longer dosing intervals, e.g. at intervals of 5 to 10 days. The pharmacokinetic properties of the anti-MIF antibodies are, in particular, also compatible with an administration of the dosages of the invention at such longer dosing intervals,

### Example 5: Tissue Distribution of anti-MIF antibodies and oxMIF in a Chronic Lymphocytic Leukemia (CLL) Model

Tissue distribution of anti-MIF antibodies and oxMIF was assessed by using a Chronic Lymphocytic Leukemia (CLL) model. In particular, a Eµ-myc lymphoma xenograft model in CD1 and C57B16 mice was conducted to assess RAM9 tissue distribution. The experiment included four treatment groups (each with 6 mice per group):

| | |
|---|---|
| - PBS | |
| - BaxC3 control antibody | 60 mg/kg |
| - RAM9 | 60 mg/kg |

The tumors were allowed to establish for 5 days. The animals were treated every other day for up to 15 days depending on survival time. Tumors (lymph nodes) were resected after the treatment period, snap frozen and embedded for tissue section preparation. Tissue sections were stained for RAM9 tissue penetration and for oxMIF. Full slides were scanned by an Olympus slide scanning microscope (VS120). Digital full slide images were analyzed using Definiens Tissue Studio™ to compare:
- RAM9 and oxMIF distribution (stained area) in the PBS and RAM9 treated groups
- Distribution of human IgG in all treatment groups

IHC staining was detected/marked up by digital image analysis in order to do semi-quantitative calculation (% marker area = % stained area). The results of the quantitation and the results of the tissue stainings are shown in Figure 3. As can be seen from the sample of the PBS-treated negative control treatment group that was stained for the presence of the RAM9 antibody (sample 1), no RAM9 antibody was detected in this sample. In samples 2 and 3 oxMIF has been detected after treatment with PBS (control) or RAM9. The tumor tissue shows approx. 6 % marker area, irrespective of the RAM9 treatment. Sample 4 shows RAM9 detection in the tumor tissue after RAM9 treatment. The marker area is approx. 3fold higher than for oxMIF (samples 2 and 3) showing that 3 times more antibody is present in the tissue as would be required for oxMIF neutralization.

Thus, these data show that a dosage of 60 mg/kg RAM9 antibody leads to clear tumor tissue penetration. Furthermore, these data demonstrate that the RAM9 antibody in the tumor tissue exceeded the amount of the target (oxMIF). Results from example 5 show, that at 60 mg/kg dose in mice target saturation can be reached despite of the short half-life of the antibody. This experiment supports the application of an efficacious lower dose in humans.

### Conclusions

Based on the findings of the present inventors, the most effective treatment of solid cancer tumors with anti-MIF antibodies in mice can be carried out with lower dosages in the range of 15 to 100 mg/kg q2d rather than with a high dosage of the anti-MIF antibodies. In view of these findings, the present inventors made an effort to determine a most effective dosage for administration of anti-MIF antibodies to humans in a series of experiments. In this regard, firstly, the inventors performed *in vitro* experiments in order to address potency *in vitro.* These experiments surprisingly indicated that anti-MIF antibodies are about 10-fold more potent in human *in vitro* assays than in rodent *in vitro* assays. Secondly, the inventors found that compared to the results obtained in mice, the anti-MIF antibodies exhibit much more favorable pharmacokinetic properties when administered to primates. Finally, the inventors also found that the anti-oxMIF antibodies exhibit a high degree of target saturation.

Taken together, based on the surprising results of the *in vitro* potency experiments, the most preferred dosage of anti-MIF antibodies in humans is about 10-fold lower than in mice. This is supported by the tissue distribution experiments. In addition, the dose response curve is non-monotonic and the dose showing an optimum efficacy lies for human application in the range of 1-10 mg/kg. Accordingly, the present invention provides an improved administration of anti-MIF antibodies to humans at the dosage ranges and dosages as indicated in the preferred embodiments.

## Claims

1. An anti-MIF antibody for use in the treatment of cancer in a human subject, wherein the anti-MIF antibody is to be administered intravenously to the human subject at a dosage in the range of 1 mg per kg body weight to 10 mg per kg body weight, and wherein the cancer to be treated is a solid tumor.

2. The anti-MIF antibody according to claim 1 for the use according to claim 1, wherein the anti-MIF antibody is to be administered to the human subject at a dosage in the range of 2.5 mg per kg body weight to 8 mg per kg body weight, at a dosage in the range of 4.5 mg per kg body weight to 5.5 mg per kg body weight, preferably at a dosage of 5 mg per kg body weight, optionally at a dosage of 2.5, 4, 6, 7.5, 8, or 10 mg per kg body weight.

3. The anti-MIF antibody according to claim 1 or 2 for the use according to claim 1 or 2, wherein the cancer is selected from the following group: pancreatic cancer, ovarian cancer, prostate cancer, breast cancer, colorectal cancer, lung cancer and colon cancer, more preferred pancreatic cancer, colorectal cancer, prostate cancer, non-small cell lung cancer and ovarian cancer, most preferred colorectal cancer, non-small cell lung cancer and ovarian cancer.

4. The anti-MIF antibody according to any one of claims 1 to 3 for the use according to any one of claims 1 to 3, wherein the anti-MIF antibody is to be administered to the human subject at a dosage in the range of 5.5 mg per kg body weight to 6.5 mg per kg body weight, preferably at a dosage of 6 mg per kg body weight, and wherein the cancer is pancreatic cancer or ovarian cancer; optionally, wherein the anti-MIF antibody is to be administered to the human subject at a dosage in the range of 2.5 mg per kg body weight to 8 mg per kg body weight, preferably at a dosage in the range of 3 mg per kg body weight to 4 mg per kg body weight, and wherein the cancer is prostate cancer; further optionally, wherein the anti-MIF antibody is to be administered to the human subject at a dosage of 2.5 mg per kg body weight, and wherein the cancer is selected from the following group: colorectal cancer, non-small cell lung cancer and ovarian cancer; further optionally, wherein the anti-MIF antibody is to be administered to the human subject at a dosage of 4, 5, 6, 7.5, 8 or 10 mg per kg body weight, and wherein the cancer is selected from the following group: colorectal cancer, non-small cell lung cancer and ovarian cancer.

5. The anti-MIF antibody according to any of the preceding claims for the use according to any of the preceding claims, wherein the dosage is to be administered every 5 to 10 days for the duration of the treatment, optionally, wherein the dosage is to be administered every 7 days for the duration of the treatment.

6. The anti-MIF antibody according to any one of claims 1 to 5 for the use of any one of claims 1 to 5, wherein the anti-MIF antibody is selected from the following group:
an anti-MIF antibody having a heavy chain encoded by the plasmid deposited with the DSMZ, Braunschweig, Germany in *E.coli* under the Budapest Treaty under the accession number DSM 25860 and a light chain encoded by the plasmid deposited with the DSMZ, Braunschweig, Germany in *E.coli* under the Budapest Treaty under the accession number DSM 25859,
an anti-MIF antibody having a heavy chain encoded by the plasmid deposited with the DSMZ, Braunschweig, Germany in *E.coli* under the Budapest Treaty under the accession number DSM 25864 and a light chain encoded by the plasmid deposited with the DSMZ, Braunschweig, Germany in *E.coli* under the Budapest Treaty under the accession number DSM 25863and/or
an anti-MIF antibody having a heavy chain encoded by the plasmid deposited with the DSMZ, Braunschweig, Germany in *E.coli* under the Budapest Treaty under the accession number DSM 25862 and a light chain encoded by the plasmid deposited with the DSMZ, Braunschweig, Germany in *E.coli* under the Budapest Treaty under the accession number DSM 25861.

7. The antibody of any one of claims 1 to 6 for the use of any one of claims 1 to 6, wherein the antibody is formulated in form of a pharmaceutically acceptable composition.

8. The antibody of any one of claims 1 to 7 for the use of any one of claims 1 to 7, wherein the use is a use in combination with a chemotherapeutic agent.

9. The antibody of any one of claims 1 to 8 for the use of any one of claims 1 to 8, wherein the use is a use in combination with panitumumab.

10. The antibody of claim 9 for the use of claim 9, wherein the cancer is colorectal cancer, preferably, wherein the colorectal cancer is metastatic colorectal cancer, and wherein the treatment is a third-line therapy or a fourth-line therapy.

11. The antibody of claim 8 for the use of claim 8, wherein the chemotherapeutic agent is a combination of 5-fluorouracil and leucovorin.

12. The antibody of claim 11 for the use of claim 11, wherein the cancer is colorectal cancer, and preferably, wherein the colorectal cancer is metastatic colorectal cancer, and wherein the treatment is a third-line therapy or a fourth-line therapy, even further preferably wherein the anti-MIF antibody is to be administered to the human subject at a dosage of 7.5 mg per kg body weight or at a dosage of 10 mg per kg body weight.

13. The antibody of anyone of claims 1 to 12 for the use of anyone of claims 1 to 12, wherein the anti-MIF antibody is the anti-MIF antibody having a heavy chain encoded by the plasmid deposited with the DSMZ, Braunschweig, Germany in *E.coli* under the Budapest Treaty under the accession number DSM 25860 and a light chain encoded by the plasmid deposited with the DSMZ, Braunschweig, Germany in *E.coli* under the Budapest Treaty under the accession number DSM 25859.

14. A kit comprising the anti-MIF antibody dosages as defined in any of claims 1 - 13 above and instructions for use, and optionally further comprising the chemotherapeutic agent as defined in claim 8 or 11, preferably further comprising panitumumab as defined in claim 9.

## Patentansprüche

1. Anti-MIF-Antikörper zur Verwendung bei der Behandlung von Krebs in einem menschlichen Subjekt, wobei der Anti-MIF-Antikörper dem menschlichen Subjekt in einer Dosis im Bereich von 1 mg pro kg Körpergewicht bis 10 mg pro kg Körpergewicht intravenös zu verabreichen ist, und wobei der zu behandelnde Krebs ein fester Tumor ist.

2. Anti-MIF-Antikörper gemäß Anspruch 1 zur Verwendung gemäß Anspruch 1, wobei der Anti-MIF-Antikörper dem menschlichen Subjekt in einer Dosis im Bereich von 2,5 mg pro kg Körpergewicht bis 8 mg pro kg Körpergewicht, in einer Dosis im Bereich von 4,5 mg pro kg Körpergewicht bis 5,5 mg pro kg Körpergewicht, bevorzugt in einer Dosis von 5 mg pro kg Körpergewicht, optional in einer Dosis von 2,5, 4, 6, 7,5, 8 oder 10 mg pro kg Körpergewicht zu verabreichen ist.

3. Anti-MIF-Antikörper gemäß Anspruch 1 oder 2 zur Verwendung gemäß Anspruch 1 oder 2, wobei der Krebs aus der folgenden Gruppe ausgewählt ist: Pankreaskrebs, Eierstockkrebs, Prostatakrebs, Brustkrebs, Kolorektalkrebs, Lungenkrebs und Darmkrebs, bevorzugter Pankreaskrebs, Kolorektalkrebs, Prostatakrebs, nicht-kleinzelliger Lungenkrebs und Eierstockkrebs, am meisten bevorzugt Kolorektalkrebs, nicht-kleinzelliger Lungenkrebs und Eierstockkrebs.

4. Anti-MIF-Antikörper gemäß mindestens einem der Ansprüche 1 bis 3 zur Verwendung gemäß mindestens einem der Ansprüche 1 bis 3, wobei der Anti-MIF-Antikörper dem menschlichen Subjekt in einer Dosis im Bereich von 5,5 mg pro kg Körpergewicht bis 6,5 mg pro kg Körpergewicht, bevorzugt in einer Dosis von 6 mg pro kg Körpergewicht, zu verabreichen ist, und wobei der Krebs Pankreaskrebs oder Eierstockkrebs ist; wobei der Anti-MIF-Antikörper optional dem menschlichen Subjekt in einer Dosis im Bereich von 2,5 mg pro kg Körpergewicht bis 8 mg pro kg Körpergewicht, bevorzugt in einer Dosis im Bereich von 3 mg pro kg Körpergewicht bis 4 mg pro kg Körpergewicht, zu verabreichen ist, und wobei der Krebs Prostatakrebs ist; wobei der Anti-MIF-Antikörper weiterhin optional dem menschlichen Subjekt in einer Dosis von 2,5 mg pro kg Körpergewicht zu verabreichen ist, und wobei der Krebs aus der folgenden Gruppe ausgewählt ist:
Kolorektalkrebs, nicht-kleinzelliger Lungenkrebs und Eierstockkrebs; wobei der Anti-MIF-Antikörper weiterhin optional dem menschlichen Subjekt in einer Dosis von 4, 5, 6, 7,5, 8 oder 10 mg pro kg Körpergewicht zu verabreichen ist, und wobei der Krebs aus der folgenden Gruppe ausgewählt ist: Kolorektalkrebs, nicht-kleinzelliger Lungenkrebs und Eierstockkrebs.

5. Anti-MIF-Antikörper gemäß irgendeinem der vorangegangenen Ansprüche zur Verwendung gemäß irgendeinem der vorangegangenen Ansprüche, wobei die Dosierung für die Dauer der Behandlung alle 5 bis 10 Tage zu verabreichen ist, wobei die Dosierung optional für die Dauer der Behandlung alle 7 Tage zu verabreichen ist.

6. Anti-MIF-Antikörper gemäß mindestens einem der Ansprüche 1 bis 5 zur Verwendung nach mindestens einem der Ansprüche 1 bis 5, wobei der Anti-MIF-Antikörper aus der folgenden Gruppe ausgewählt ist:
einem Anti-MIF-Antikörper mit einer schweren Kette, codiert durch das Plasmid, hinterlegt bei der DSMZ, Braunschweig, Deutschland, in *E. coli* unter dem Budapester Vertrag unter der Hinterlegungsnummer DSM 25860, und einer leichten Kette, codiert durch das Plasmid, hinterlegt bei der DSMZ, Braunschweig, Deutschland in *E. coli* unter dem Budapester Vertrag unter der Hinterlegungsnummer DSM 25859,
einem Anti-MIF-Antikörper mit einer schweren Kette, codiert durch das Plasmid, hinterlegt bei der DSMZ, Braunschweig, Deutschland in *E. coli* unter dem Budapester Vertrag unter der Hinterlegungsnummer DSM 25864, und einer leichten Kette, codiert durch das Plasmid, hinterlegt bei der DSMZ, Braunschweig, Deutschland in *E. coli* unter dem Budapester Vertrag unter der Hinterlegungsnummer DSM 25863, und/oder
einem Anti-MIF-Antikörper mit einer schweren Kette, codiert durch das Plasmid, hinterlegt bei der DSMZ, Braunschweig, Deutschland in *E. coli* unter dem Budapester Vertrag unter der Hinterlegungsnummer DSM 25862, und einer leichten Kette, codiert durch das Plasmid, hinterlegt bei der DSMZ, Braunschweig, Deutschland in *E. coli* unter dem Budapester Vertrag unter der Hinterlegungsnummer DSM 25861.

7. Antikörper nach mindestens einem der Ansprüche 1 bis 6 zur Verwendung nach mindestens einem der Ansprüche 1 bis 6, wobei der Antikörper in Form einer pharmazeutisch akzeptablen Zusammensetzung formuliert ist.

8. Antikörper nach mindestens einem der Ansprüche 1 bis 7 zur Verwendung nach mindestens einem der Ansprüche 1 bis 7, wobei die Verwendung eine Verwendung in Kombination mit einem chemotherapeutischen Agens ist.

9. Antikörper nach mindestens einem der Ansprüche 1 bis 8 zur Verwendung nach mindestens einem der Ansprüche 1 bis 8, wobei die Verwendung eine Verwendung in Kombination mit Panitumumab ist.

10. Antikörper nach Anspruch 9 zur Verwendung nach Anspruch 9, wobei der Krebs Kolorektalkrebs ist, wobei der Kolorektalkrebs bevorzugt metastasierender Kolorektalkrebs ist, und wobei die Behandlung eine Drittlinientherapie oder eine Viertlinientherapie ist.

11. Antikörper nach Anspruch 8 zur Verwendung nach Anspruch 8, wobei das chemotherapeutische Agens eine Kombination aus 5-Fluoruracil und Leucovorin ist.

12. Antikörper nach Anspruch 11 zur Verwendung nach Anspruch 11, wobei der Krebs Kolorektalkrebs ist und wobei der Kolorektalkrebs bevorzugt metastasierender Kolorektalkrebs ist, und wobei die Behandlung eine Drittlinientherapie oder eine Viertlinientherapie ist, wobei der Anti-MIF-Antikörper noch weiterhin bevorzugt dem menschlichen Subjekt in einer Dosis von 7,5 mg pro kg Körpergewicht oder in einer Dosis von 10 mg pro kg Körpergewicht zu verabreichen ist.

13. Antikörper nach mindestens einem der Ansprüche 1 bis 12 zur Verwendung nach mindestens einem der Ansprüche 1 bis 12, wobei der Anti-MIF-Antikörper der Anti-MIF-Antikörper mit einer schweren Kette, codiert durch das Plasmid, hinterlegt bei der DSMZ, Braunschweig, Deutschland in *E. coli* unter dem Budapester Vertrag unter der Hinterlegungsnummer DSM 25860, und einer leichten Kette, codiert durch das Plasmid, hinterlegt bei der DSMZ, Braunschweig, Deutschland in *E. coli* unter dem Budapester Vertrag unter der Hinterlegungsnummer DSM 25859, ist.

14. Kit, umfassend die Anti-MIF-Antikörper-Dosierungen wie in irgendeinem der Ansprüche 1-13 oben definiert und Anweisungen zur Verwendung, und optional weiterhin umfassend das chemotherapeutische Agens wie in Anspruch 8 oder 11 definiert, bevorzugt weiterhin umfassend Panitumumab wie in Anspruch 9 definiert.

## Revendications

1. Anticorps anti-MIF destiné à être utilisé dans le traitement du cancer chez un sujet humain, dans lequel l'anticorps anti-MIF doit être administré par voie intraveineuse au sujet humain à une posologie dans la plage de 1 mg par kg de poids corporel à 10 mg par kg de poids corporel, et dans lequel le cancer à traiter est une tumeur solide.

2. Anticorps anti-MIF selon la revendication 1 destiné à être utilisé selon la revendication 1, dans lequel l'anticorps anti-MIF doit être administré au sujet humain à une posologie dans la plage de 2,5 mg par kg de poids corporel à 8 mg par kg de poids corporel, à une posologie dans la plage de 4,5 mg par kg de poids corporel à 5,5 mg par kg de poids corporel, de préférence à une posologie de 5 mg par kg de poids corporel, facultativement à une posologie de 2,5 mg, 4, 6, 7,5, 8 ou 10 par kg de poids corporel.

3. Anticorps anti-MIF selon la revendication 1 ou 2 destiné à être utilisé selon la revendication 1 ou 2, dans lequel le cancer est sélectionné dans le groupe suivant : le cancer du pancréas, le cancer de l'ovaire, le cancer de la prostate, le cancer du sein, le cancer colorectal, le cancer du poumon et le cancer du côlon, plus préférentiellement le cancer du pancréas, le cancer colorectal, le cancer de la prostate, le cancer du poumon non à petites cellules et le cancer de l'ovaire, le plus préférentiellement, le cancer colorectal, le cancer du poumon non à petites cellules et le cancer de l'ovaire.

4. Anticorps anti-MIF selon l'une quelconque des revendications 1 à 3 destiné à être utilisé selon l'une quelconque des revendications 1 à 3, dans lequel l'anticorps anti-MIF doit être administré au sujet humain à une posologie dans la plage de 5,5 mg par kg de poids corporel à 6,5 mg par kg de poids corporel, de préférence à une posologie de 6 mg par kg de poids corporel, et dans lequel le cancer est le cancer pancréatique ou le cancer de l'ovaire ; facultativement, dans lequel l'anticorps anti-MIF doit être administré au sujet humain à une posologie dans la plage de 2,5 mg par kg de poids corporel à 8 mg par kg de poids corporel, de préférence à une posologie dans la plage de 3 mg par kg de poids corporel à 4 mg par kg de poids corporel, et dans lequel le cancer est le cancer de la prostate ; en outre facultativement, dans lequel l'anticorps anti-MIF doit être administré au sujet humain à une posologie de 2,5 mg par kg de poids corporel, et dans lequel le cancer est sélectionné dans le groupe suivant : le cancer colorectal, le cancer du poumon non à petites cellules et le cancer de l'ovaire ; en outre facultativement, dans lequel l'anticorps anti-MIF doit être administré au sujet humain à une posologie de 4, 5, 6, 7,5, 8 ou 10 mg par kg de poids corporel, et dans lequel le cancer est sélectionné dans le groupe suivant : le cancer colorectal, le cancer du poumon non à petites cellules et le cancer de l'ovaire.

5. Anticorps anti-MIF selon l'une quelconque des revendications précédentes destiné à être utilisé selon l'une quelconque des revendications précédentes, dans lequel la posologie doit être administrée tous les 5 à 10 jours pendant la durée du traitement, facultativement dans lequel la posologie doit être administrée tous les 7 jours pendant la durée du traitement.

6. Anticorps anti-MIF selon l'une quelconque des revendications 1 à 5 destiné à être utilisé selon l'une quelconque des revendications 1 à 5, dans lequel l'anticorps anti-MIF est sélectionné dans le groupe suivant :
un anticorps anti-MIF présentant une chaîne lourde codée par le plasmide déposé avec le DSMZ, Braunschweig, Allemagne, dans *E. Coli* selon le traité de Budapest sous le numéro d'accès DSM 25860 et une chaîne légère codée par le plasmide déposé avec le DSMZ, Braunschweig, Allemagne, dans *E. Coli* selon le traité de Budapest sous le numéro d'accès DSM 25859,
un anticorps anti-MIF présentant une chaîne lourde codée par le plasmide déposé avec le DSMZ, Braunschweig, Allemagne, dans *E. Coli* selon le traité de Budapest sous le numéro d'accès DSM 25864 et une chaîne légère codée par le plasmide déposé avec le DSMZ, Braunschweig, Allemagne, dans *E. Coli* selon le traité de Budapest sous le numéro d'accès DSM 25863 et/ou
un anticorps anti-MIF présentant une chaîne lourde codée par le plasmide déposé avec le DSMZ, Braunschweig, Allemagne, dans *E. Coli* selon le traité de Budapest sous le numéro d'accès DSM 25862 et une chaîne légère codée par le plasmide déposé avec le DSMZ, Braunschweig, Allemagne, dans *E. Coli* selon le traité de Budapest sous le numéro d'accès DSM 25861.

7. Anticorps anti-MIF selon l'une quelconque des revendications 1 à 6 destiné à être utilisé selon l'une quelconque des revendications 1 à 6, dans lequel l'anticorps est formulé sous la forme d'une composition pharmaceutiquement acceptable.

8. Anticorps anti-MIF selon l'une quelconque des revendications 1 à 7 destiné à être utilisé selon l'une quelconque des revendications 1 à 7, dans lequel l'utilisation est une utilisation en combinaison avec un agent chimiothérapeutique.

9. Anticorps anti-MIF selon l'une quelconque des revendications 1 à 8 destiné à être utilisé selon l'une quelconque des revendications 1 à 8, dans lequel l'utilisation est une utilisation en combinaison avec le panitumumab.

10. Anticorps anti-MIF selon la revendication 9 destiné à être utilisé selon la revendication 9, dans lequel le cancer est le cancer colorectal, de préférence dans lequel le cancer colorectal est un cancer colorectal métastatique, et dans lequel le traitement est une thérapie de troisième intention ou une thérapie de quatrième intention.

11. Anticorps anti-MIF selon la revendication 8 destiné à être utilisé selon la revendication 8, dans lequel l'agent chimiothérapeutique est une combinaison de 5-fluorouracil et de leucovorine.

12. Anticorps anti-MIF selon la revendication 11 destiné à être utilisé selon la revendication 11, dans lequel le cancer est le cancer colorectal et, de préférence, dans lequel le cancer colorectal est un cancer colorectal métastatique, et dans lequel le traitement est une thérapie de troisième intention ou une thérapie de quatrième intention, encore plus préférentiellement dans lequel l'anticorps anti-MF doit être administré au sujet humain à une posologie de 7,5 mg par kg de poids corporel ou à une posologie de 10 mg par kg de poids corporel.

13. Anticorps anti-MIF selon l'une quelconque des revendications 1 à 12 destiné à être utilisé selon l'une quelconque des revendications 1 à 12, dans lequel l'anticorps anti-MIF est l'anticorps anti-MIF présentant une chaîne lourde codée par le plasmide déposé avec le DSMZ, Braunschweig, Allemagne, dans *E. Coli* selon le traité de Budapest sous le numéro d'accès DSM 25860 et une chaîne légère codée par le plasmide déposé avec le DSMZ, Braunschweig, Allemagne, dans *E. Coli* selon le traité de Budapest sous le numéro d'accès DSM 25859.

14. Kit comprenant les posologies d'anticorps anti-MIF telles que définies dans l'une quelconque des revendications 1-13 ci-dessus et instructions destinées à être utilisées, et facultativement, comprenant en outre l'agent chimiothérapeutique tel que défini dans la revendication 8 ou 11, de préférence comprenant en outre du panitumumab tel que défini dans la revendication 9.


### SEQUENCE LISTING

<110> Baxalta Incorporated
   Baxalta GmbH
<120> DOSAGE REGIMEN FOR ANTI-MIF ANTIBODIES
<130> 188 439
<150> US 62/141,190
   <151> 2015-03-31
<150> US 62/163,267
   <151> 2015-05-18
<160> 14
<170> PatentIn version 3.5
<210> 1
   <211> 214
   <212> PRT
   <213> Artificial Sequence
<220>
   <223> Light chain of RAB9
<400> 1
   Asp Ile Gln Met Thr Gln Ser Pro Ser Ser Leu Ser Ala Ser Val Gly
   1 5 10 15
   Asp Arg Val Thr Ile Thr Cys Arg Ser Ser Gln Arg Ile Met Thr Tyr
   20 25 30
   Leu Asn Trp Tyr Gln Gln Lys Pro Gly Lys Ala Pro Lys Leu Leu Ile
   35 40 45
   Phe Val Ala Ser His Ser Gln Ser Gly Val Pro Ser Arg Phe Arg Gly
   50 55 60
   Ser Gly Ser Glu Thr Asp Phe Thr Leu Thr Ile Ser Gly Leu Gln Pro
   65 70 75 80
   Glu Asp Ser Ala Thr Tyr Tyr Cys Gln Gln Ser Phe Trp Thr Pro Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 2
   <211> 214
   <212> PRT
   <213> Artificial Sequence
<220>
   <223> Light chain of RAB4
<400> 2
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 214
   <212> PRT
   <213> Artificial Sequence
<220>
   <223> Light chain of RAB0
<400> 3
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 4
   <211> 214
   <212> PRT
   <213> Artificial Sequence
<220>
   <223> Light chain of RAB2
<400> 4
   Asp Ile Gln Met Thr Gln Ser Pro Val Thr Leu Ser Leu Ser Pro Gly
   1               5                   10                  15
   Glu Arg Ala Thr Leu Ser Cys Arg Ala Ser Gln Ser Val Arg Ser Ser
               20                  25                  30
   Tyr Leu Ala Trp Tyr Gln Gln Lys Pro Gly Gln Thr Pro Arg Leu Leu
           35                  40                  45
   Ile Tyr Gly Ala Ser Asn Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Asn Ser Leu
                   85                  90                  95
   Thr Phe Gly Gly Gly Thr Lys Val Glu Ile Lys Arg Thr Val Ala Ala
                   100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 5
   <211> 445
   <212> PRT
   <213> Artificial Sequence
<220>
   <223> Heavy chain of RAB9
<400> 5
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ser Met Asn Trp Val Arg Gln Ala Pro Gly Lys Gly Leu Glu Trp Val
           35                  40                  45
   Ser Ser Ile Gly Ser Ser Gly Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Gly Ser Gln Trp Leu Tyr Gly Met Asp Val Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Lys Thr Tyr Thr Cys Asn Val Asp His Lys Pro Ser
           195                 200                 205
   Asn Thr Lys Val Asp Lys Arg Val Glu Ser Lys Tyr Gly Pro Pro Cys
       210                 215                 220
   Pro Pro Cys Pro Ala Pro Glu Phe Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser Gln Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Gly Leu Pro Ser Ser Ile Glu Lys Thr Ile Ser Lys
                   325                 330                 335
   Ala Lys Gly Gln Pro Arg Glu Pro Gln Val Tyr Thr Leu Pro Pro Ser
   340 345 350
   Gln Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys
   435 440 445
<210> 6
   <211> 454
   <212> PRT
   <213> Artificial Sequence
<220>
   <223> Heavy chain of RAB4
<400> 6
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Asp Trp Val Arg Gln Ala Pro Gly Lys Gly Leu Glu Trp Val
           35                  40                  45
   Ser Gly Ile Val Pro Ser Gly Gly Phe Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
   115 120 125
   Ser Thr Lys Gly Pro Ser Val Phe Pro Leu Ala Pro Cys Ser Arg Ser
   130 135 140
   Thr Ser Glu Ser Thr Ala Ala Leu Gly Cys Leu Val Lys Asp Tyr Phe
   145 150 155 160
   Pro Glu Pro Val Thr Val Ser Trp Asn Ser Gly Ala Leu Thr Ser Gly
   165 170 175
   Val His Thr Phe Pro Ala Val Leu Gln Ser Ser Gly Leu Tyr Ser Leu
   180 185 190
   Ser Ser Val Val Thr Val Pro Ser Ser Ser Leu Gly Thr Lys Thr Tyr
   195 200 205
   Thr Cys Asn Val Asp His Lys Pro Ser Asn Thr Lys Val Asp Lys Arg
   210 215 220
   Val Glu Ser Lys Tyr Gly Pro Pro Cys Pro Pro Cys Pro Ala Pro Glu
   225 230 235 240
   Phe Leu Gly Gly Pro Ser Val Phe Leu Phe Pro Pro Lys Pro Lys Asp
   245 250 255
   Thr Leu Met Ile Ser Arg Thr Pro Glu Val Thr Cys Val Val Val Asp
   260 265 270
   Val Ser Gln Glu Asp Pro Glu Val Gln Phe Asn Trp Tyr Val Asp Gly
   275 280 285
   Val Glu Val His Asn Ala Lys Thr Lys Pro Arg Glu Glu Gln Phe Asn
   290 295 300
   Ser Thr Tyr Arg Val Val Ser Val Leu Thr Val Leu His Gln Asp Trp
   305 310 315 320
   Leu Asn Gly Lys Glu Tyr Lys Cys Lys Val Ser Asn Lys Gly Leu Pro
   325 330 335
   Ser Ser Ile Glu Lys Thr Ile Ser Lys Ala Lys Gly Gln Pro Arg Glu
   340 345 350
   Pro Gln Val Tyr Thr Leu Pro Pro Ser Gln Glu Glu Met Thr Lys Asn
                   355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Arg
                   405                 410                 415
   Leu Thr Val Asp Lys Ser Arg Trp Gln Glu Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Leu Gly Lys
       450
<210> 7
   <211> 454
   <212> PRT
   <213> Artificial Sequence
<220>
   <223> Heavy chain of RAB0
<400> 7
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Trp Tyr
               20                  25                  30
   Ala Met Asp Trp Val Arg Gln Ala Pro Gly Lys Gly Leu Glu Trp Val
           35                  40                  45
   Ser Gly Ile Tyr Pro Ser Gly Gly Arg Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
   115 120 125
   Ser Thr Lys Gly Pro Ser Val Phe Pro Leu Ala Pro Cys Ser Arg Ser
   130 135 140
   Thr Ser Glu Ser Thr Ala Ala Leu Gly Cys Leu Val Lys Asp Tyr Phe
   145 150 155 160
   Pro Glu Pro Val Thr Val Ser Trp Asn Ser Gly Ala Leu Thr Ser Gly
   165 170 175
   Val His Thr Phe Pro Ala Val Leu Gln Ser Ser Gly Leu Tyr Ser Leu
   180 185 190
   Ser Ser Val Val Thr Val Pro Ser Ser Ser Leu Gly Thr Lys Thr Tyr
   195 200 205
   Thr Cys Asn Val Asp His Lys Pro Ser Asn Thr Lys Val Asp Lys Arg
   210 215 220
   Val Glu Ser Lys Tyr Gly Pro Pro Cys Pro Pro Cys Pro Ala Pro Glu
   225 230 235 240
   Phe Leu Gly Gly Pro Ser Val Phe Leu Phe Pro Pro Lys Pro Lys Asp
   245 250 255
   Thr Leu Met Ile Ser Arg Thr Pro Glu Val Thr Cys Val Val Val Asp
   260 265 270
   Val Ser Gln Glu Asp Pro Glu Val Gln Phe Asn Trp Tyr Val Asp Gly
   275 280 285
   Val Glu Val His Asn Ala Lys Thr Lys Pro Arg Glu Glu Gln Phe Asn
   290 295 300
   Ser Thr Tyr Arg Val Val Ser Val Leu Thr Val Leu His Gln Asp Trp
   305 310 315 320
   Leu Asn Gly Lys Glu Tyr Lys Cys Lys Val Ser Asn Lys Gly Leu Pro
   325 330 335
   Ser Ser Ile Glu Lys Thr Ile Ser Lys Ala Lys Gly Gln Pro Arg Glu
   340 345 350
   Pro Gln Val Tyr Thr Leu Pro Pro Ser Gln Glu Glu Met Thr Lys Asn
   355 360 365
   Gln Val Ser Leu Thr Cys Leu Val Lys Gly Phe Tyr Pro Ser Asp Ile
   370 375 380
   Ala Val Glu Trp Glu Ser Asn Gly Gln Pro Glu Asn Asn Tyr Lys Thr
   385 390 395 400
   Thr Pro Pro Val Leu Asp Ser Asp Gly Ser Phe Phe Leu Tyr Ser Arg
   405 410 415
   Leu Thr Val Asp Lys Ser Arg Trp Gln Glu Gly Asn Val Phe Ser Cys
   420 425 430
   Ser Val Met His Glu Ala Leu His Asn His Tyr Thr Gln Lys Ser Leu
   435 440 445
   Ser Leu Ser Leu Gly Lys
   450
<210> 8
   <211> 454
   <212> PRT
   <213> Artificial Sequence
<220>
   <223> Heavy chain of RAB2
<400> 8
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Asp Trp Val Arg Gln Ala Pro Gly Lys Gly Leu Glu Trp Val
           35                  40                  45
   Ser Gly Ile Val Pro Ser Gly Gly Phe Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
           115                 120                 125
   Ser Thr Lys Gly Pro Ser Val Phe Pro Leu Ala Pro Cys Ser Arg Ser
       130                 135                 140
   Thr Ser Glu Ser Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Lys Thr Tyr
           195                 200                 205
   Thr Cys Asn Val Asp His Lys Pro Ser Asn Thr Lys Val Asp Lys Arg
       210                 215                 220
   Val Glu Ser Lys Tyr Gly Pro Pro Cys Pro Pro Cys Pro Ala Pro Glu
   225                 230                 235                 240
   Phe Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser Gln Glu Asp Pro Glu Val Gln Phe Asn Trp Tyr Val Asp Gly
           275                 280                 285
   Val Glu Val His Asn Ala Lys Thr Lys Pro Arg Glu Glu Gln Phe Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Gly Leu Pro
                   325                 330                 335
   Ser Ser Ile Glu Lys Thr Ile Ser Lys Ala Lys Gly Gln Pro Arg Glu
               340                 345                 350
   Pro Gln Val Tyr Thr Leu Pro Pro Ser Gln Glu Glu Met Thr Lys Asn
                   355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Arg
                   405                 410                 415
   Leu Thr Val Asp Lys Ser Arg Trp Gln Glu Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Leu Gly Lys
       450
<210> 9
   <211> 457
   <212> PRT
   <213> Artificial Sequence
<220>
   <223> RAM0hc
<400> 9
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Trp Tyr
               20                  25                  30
   Ala Met Asp Trp Val Arg Gln Ala Pro Gly Lys Gly Leu Glu Trp Val
           35                  40                  45
   Ser Gly Ile Tyr Pro Ser Gly Gly Arg Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Arg
       210                 215                 220
   Val Glu Pro Lys Ser Cys Asp Lys Thr His Thr Cys Pro Pro Cys Pro
   225                 230                 235                 240
   Ala Pro Glu Leu Leu Gly Gly Pro Ser Val Phe Leu Phe Pro Pro Lys
                   245                 250                 255
   Pro Lys Asp Thr Leu Met Ile Ser Arg Thr Pro Glu Val Thr Cys Val
               260                 265                 270
   Val Val Asp Val Ser His Glu Asp Pro Glu Val Lys Phe Asn Trp Tyr
           275                 280                 285
   Val Asp Gly Val Glu Val His Asn Ala Lys Thr Lys Pro Arg Glu Glu
       290                 295                 300
   Gln Tyr Asn Ser Thr Tyr Arg Val Val Ser Val Leu Thr Val Leu His
   305                 310                 315                 320
   Gln Asp Trp Leu Asn Gly Lys Glu Tyr Lys Cys Lys Val Ser Asn Lys
                   325                 330                 335
   Ala Leu Pro Ala Pro Ile Glu Lys Thr Ile Ser Lys Ala Lys Gly Gln
               340                 345                 350
   Pro Arg Glu Pro Gln Val Tyr Thr Leu Pro Pro Ser Arg Glu Glu Met
               355                 360                 365
   Thr Lys Asn Gln Val Ser Leu Thr Cys Leu Val Lys Gly Phe Tyr Pro
       370                 375                 380
   Ser Asp Ile Ala Val Glu Trp Glu Ser Asn Gly Gln Pro Glu Asn Asn
   385                 390                 395                 400
   Tyr Lys Thr Thr Pro Pro Val Leu Asp Ser Asp Gly Ser Phe Phe Leu
                   405                 410                 415
   Tyr Ser Lys Leu Thr Val Asp Lys Ser Arg Trp Gln Gln Gly Asn Val
               420                 425                 430
   Phe Ser Cys Ser Val Met His Glu Ala Leu His Asn His Tyr Thr Gln
           435                 440                 445
   Lys Ser Leu Ser Leu Ser Pro Gly Lys
       450                 455
<210> 10
   <211> 214
   <212> **PRT**
   <213> **Artificial** Sequence
<220>
   <223> RAM01c
<400> 10
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 11
   <211> **448**
   <212> **PRT**
   <213> Artificial Sequence
<220>
   <223> RAM9hc
<400> 11
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ser Met Asn Trp Val Arg Gln Ala Pro Gly Lys Gly Leu Glu Trp Val
           35                  40                  45
   Ser Ser Ile Gly Ser Ser Gly Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Gly Ser Gln Trp Leu Tyr Gly Met Asp Val Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 12
   <211> 214
   <212> **PRT**
   <213> **Artificial** Sequence
<220>
   <223> RAM91c
<400> 12
   Asp Ile Gln Met Thr Gln Ser Pro Ser Ser Leu Ser Ala Ser Val Gly
   1 5 10 15
   Asp Arg Val Thr Ile Thr Cys Arg Ser Ser Gln Arg Ile Met Thr Tyr
   20 25 30
   Leu Asn Trp Tyr Gln Gln Lys Pro Gly Lys Ala Pro Lys Leu Leu Ile
   35 40 45
   Phe Val Ala Ser His Ser Gln Ser Gly Val Pro Ser Arg Phe Arg Gly
   50 55 60
   Ser Gly Ser Glu Thr Asp Phe Thr Leu Thr Ile Ser Gly Leu Gln Pro
   65 70 75 80
   Glu Asp Ser Ala Thr Tyr Tyr Cys Gln Gln Ser Phe Trp Thr Pro Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 13
   <211> 457
   <212> PRT
   <213> Artificial Sequence
<220>
   <223> RAM4hc
<400> 13
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Asp Trp Val Arg Gln Ala Pro Gly Lys Gly Leu Glu Trp Val
           35                  40                  45
   Ser Gly Ile Val Pro Ser Gly Gly Phe Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Arg
       210                 215                 220
   Val Glu Pro Lys Ser Cys Asp Lys Thr His Thr Cys Pro Pro Cys Pro
   225                 230                 235                 240
   Ala Pro Glu Leu Leu Gly Gly Pro Ser Val Phe Leu Phe Pro Pro Lys
                   245                 250                 255
   Pro Lys Asp Thr Leu Met Ile Ser Arg Thr Pro Glu Val Thr Cys Val
               260                 265                 270
   Val Val Asp Val Ser His Glu Asp Pro Glu Val Lys Phe Asn Trp Tyr
           275                 280                 285
   Val Asp Gly Val Glu Val His Asn Ala Lys Thr Lys Pro Arg Glu Glu
       290                 295                 300
   Gln Tyr Asn Ser Thr Tyr Arg Val Val Ser Val Leu Thr Val Leu His
   305                 310                 315                 320
   Gln Asp Trp Leu Asn Gly Lys Glu Tyr Lys Cys Lys Val Ser Asn Lys
                   325                 330                 335
   Ala Leu Pro Ala Pro Ile Glu Lys Thr Ile Ser Lys Ala Lys Gly Gln
               340                 345                 350
   Pro Arg Glu Pro Gln Val Tyr Thr Leu Pro Pro Ser Arg Glu Glu Met
   355 360 365
   Thr Lys Asn Gln Val Ser Leu Thr Cys Leu Val Lys Gly Phe Tyr Pro
   370 375 380
   Ser Asp Ile Ala Val Glu Trp Glu Ser Asn Gly Gln Pro Glu Asn Asn
   385 390 395 400
   Tyr Lys Thr Thr Pro Pro Val Leu Asp Ser Asp Gly Ser Phe Phe Leu
   405 410 415
   Tyr Ser Lys Leu Thr Val Asp Lys Ser Arg Trp Gln Gln Gly Asn Val
   420 425 430
   Phe Ser Cys Ser Val Met His Glu Ala Leu His Asn His Tyr Thr Gln
   435 440 445
   Lys Ser Leu Ser Leu Ser Pro Gly Lys
   450 455
<210> 14
   <211> 214
   <212> **PRT**
   <213> **Artificial** Sequence
<220>
   <223> RAM41c
<400> **14**
   Asp Ile Gln Met Thr Gln Ser Pro Gly Thr Leu Ser Leu Ser Pro Gly
   1 5 10 15
   Glu Arg Ala Thr Leu Ser Cys Arg Ala Ser Gln Gly Val Ser Ser Ser
   20 25 30
   Ser Leu Ala Trp Tyr Gln Gln Lys Pro Gly Gln Ala Pro Arg Leu Leu
   35 40 45
   Ile Tyr Gly Thr Ser Ser Arg Ala Thr Gly Ile Pro Asp Arg Phe Ser
   50 55 60
   Gly Ser Ala Ser Gly Thr Asp Phe Thr Leu Thr Ile Ser Arg Leu Gln
   65 70 75 80
   Pro Glu Asp Phe Ala Val Tyr Tyr Cys Gln Gln Tyr Gly Arg Ser Leu
   85 90 95
   Thr Phe Gly Gly Gly Thr Lys Val Glu Ile Lys Arg Thr Val Ala Ala
             100                 105                 110
   Pro Ser Val Phe Ile Phe Pro Pro Ser Asp Glu Gln Leu Lys Ser Gly
         115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210